(19) **Europäisches Patentamt / European Patent Office / Office européen des brevets**

(11) **EP 3 612 259 B1**

(12) # EUROPEAN PATENT SPECIFICATION

(45) Date of publication and mention
of the grant of the patent:
**09.11.2022 Bulletin 2022/45**

(21) Application number: **18722352.4**

(22) Date of filing: **17.04.2018**

(51) International Patent Classification (IPC):
*A61M 11/00* (2006.01)   *A61M 15/00* (2006.01)

(52) Cooperative Patent Classification (CPC):
(C-Sets available)
**A61M 15/0008; A61M 11/002; A61M 11/003;
A61M 15/0005; A61M 15/0028; A61M 15/003;
A61M 15/0041; A61M 15/0086;** A61M 15/0021;
A61M 2202/064; A61M 2205/581; A61M 2206/14

(Cont.)

(86) International application number:
**PCT/US2018/027982**

(87) International publication number:
**WO 2018/195086 (25.10.2018 Gazette 2018/43)**

(54) **UNIT DOSE DRY POWDER INHALER**

EINHEITSDOSIERTROCKENPULVERINHALATOR

INHALATEUR DE POUDRE SÈCHE À DOSAGE UNITAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.04.2017 US 201762486183 P**

(43) Date of publication of application:
**26.02.2020 Bulletin 2020/09**

(73) Proprietor: **Respira Therapeutics, Inc.**
**Albuquerque, New Mexico 87110 (US)**

(72) Inventors:
• **HANNON, James**
**Superior, Colorado 80027 (US)**
• **DEATON, Dan**
**Apex, North Carolina 27539 (US)**
• **LYONS, Shirley**
**Redwood City, California 94062 (US)**
• **WEERS, Jeffry**
**Half Moon Bay, California 94019 (US)**
• **CURTIS, Robert**
**Santa Fe, New Mexico 87506 (US)**
• **DONOVAN, Martin J.**
**El Paso, Texas 79936 (US)**

(74) Representative: **Gill Jennings & Every LLP**
**The Broadgate Tower**
**20 Primrose Street**
**London EC2A 2ES (GB)**

(56) References cited:
**EP-A1- 0 388 621       EP-A1- 3 138 601
WO-A1-2013/016787   US-A1- 2012 145 150
US-A1- 2013 276 783   US-A1- 2014 318 539
US-A1- 2016 199 598   US-A1- 2016 354 563
US-A9- 2015 246 189**

(52) Cooperative Patent Classification (CPC): (Cont.)

C-Sets
A61M 2202/064, A61M 2202/0007

**Description**

This application claims priority to U.S. Provisional Patent Application No. 62/486,183, filed on April 17, 2017.

BACKGROUND OF THE INVENTION

Active pharmaceutical ingredients (APIs) that are useful for treating respiratory diseases are often formulated for administration via inhalation with portable inhalers. Pulmonary drug delivery methods and compositions that effectively provide the API to the specific site in the lungs potentially serve to minimize toxic side effects, lower dosing requirements, and decrease costs. The development of such systems for pulmonary drug delivery has long been a goal of the pharmaceutical industry. Inhalation systems commonly used to deliver drugs via inhalation include nebulizers, metered dose inhalers, and dry powder inhalers.

All currently marketed dry powder inhalers rely on the patient's inspiratory efforts to introduce a medicament in a dry powder form into the lungs. To achieve good deposition of particles in the lungs, it is generally accepted that the aerodynamic diameter must be in the respirable size range between about 1 $\mu$m and 5 $\mu$m. However, fine particles of this size are highly cohesive with poor bulk powder properties (i.e., poor powder flow, fluidization, and dispersibility).

To improve bulk powder properties of dry powder aerosols, micronized particle particles are often blended with coarse lactose monohydrate carrier particles with a geometric diameter between 50 and 200 $\mu$m. The fine drug particles and coarse lactose carrier particles form an 'adhesive mixture', with the drug particles adhered to the surface of the carrier, and the bulk powder taking on the improved powder flow and fluidization properties of the carrier.

Lactose blends require a delicate balance of surface forces. The adhesive force between the drug and the carrier must be strong enough to create an adhesive mixture that maintains its structure during filling and on storage, yet weak enough to allow the drug and carrier to separate (i.e., disperse) during aerosol administration. The adhesive force between the fine drug particles and coarse carriers remains high in current marketed dry powder products with mean lung delivery efficiencies between about 10% and 30% of the nominal dose and mean interpatient variabilities in lung delivery of approximately 30% to 50%. Moreover, a large percentage of the administered dose is deposited in the upper respiratory tract (i.e., the mouth and throat) as well as in the large airways, with only a small percentage of the drug delivered into the peripheral regions of the lungs.

It is increasingly being recognized that improved delivery to the small airways could have a significant impact in improving the effectiveness of many inhaled therapeutics. For example, the small airways contribute substantially to the pathophysiology and clinical expression of disease observed in asthma and chronic obstructive pulmonary disease (COPD) patients. Delivery to the small airways may also be critical in the treatment of patients with pulmonary arterial hypertension (PAH). PAH is a chronic disease characterized by proliferation and remodeling of vascular endothelial and smooth muscle cells in the small pulmonary arteries and arterioles. This results in a physical narrowing of the arteries, and progressive increases in pulmonary vascular resistance, elevation in pulmonary artery pressure, right heart failure, and eventually, death. Therapeutics used for the treatment of PAH are vasodilators which act on specific receptors present in the pulmonary arteries. Pulmonary arteries are present in the pre-capillary portion of the pulmonary circulation. Effective delivery of drug to the pulmonary arteries requires deposition of drug particles in the small airways of the lung. As such, there is a significant unmet need for pulmonary delivery systems that improve delivery of API into the peripheral regions of the lungs (i.e., into the small airways). It is an object of the present invention to significantly improve drug delivery into the small airways.

Current marketed dry powder inhalers comprising lactose blends deposit between about 50% and 90% of the nominal dose in the upper respiratory tract (URT). The anatomy of the soft tissue in the URT is highly variable between subjects. This leads to significant variability in lung delivery. It is another object of the present invention to more effectively bypass deposition of API in the URT, thereby reducing variability in lung delivery.

Increasing lung delivery may also decrease variability in the total lung dose (TLD) or in the dose delivered to the small airways with variations in patient inspiratory flow profile. This is sometimes referred to as flow rate dependence. It is generally believed that dry powder inhalers are inherently flow rate dependent because powder dispersion depends on the inspiratory flow profile of the patient. Increasing the TLD has been demonstrated to decrease flow rate dependence in subjects using dry powder inhalers. It is a further object of the present invention to reduce flow rate dependence in drug delivery to the lungs, and in particular drug delivery to the small airways.

US 2016/0199598 A1 discloses a dry powder inhaler according to the preamble of claim 1.

BRIEF SUMMARY OF THE INVENTION

According to the invention, there is provided a dry powder inhaler according to claim 1. The dependent claims define preferred embodiments of the invention.

Embodiments described herein are directed to unit-dose inhalers that are designed to deliver consistent dosages of powdered medicament to the smaller, peripheral regions of the lungs and reduce variability of medicament delivery associated with patient breathing patterns. More specifically, dry powder inhalers described herein improve powder dispersion relative to current marketed capsule-based dry powders, which results in an increase in total lung dose, increased delivery to the peripheral regions of the lungs, and decreased dependence on the inspiratory flow profile of the subject. This enhanced powder dispersion is a result of inclusion of four distinct dispersion elements that operate according to different mechanisms. The four main elements are: (a) centrifugal forces resulting from rotation of the capsule in the capsule seat during inhalation; (b) static impaction forces resulting from impaction of particle agglomerates with a grid or screen during inhalation; (c) shear forces at the orifice entry to a dispersion chamber; and (d) dynamic impaction and turbulent forces resulting from impaction of powder agglomerates with an axially oscillating actuator within the dispersion chamber.

One specific dry powder inhaler described herein includes an inhaler base that is configured to hold a capsule containing a powdered medicament. The capsule is pierced on either end by depressing two spring loaded buttons that each bring forward metal piercing needles. The needles pierce a single hole on either side of the capsule. The base also contains a path for airflow into the device. Airflow through the capsule seat lifts the capsule and results in precession at high speed around its main axis within a raceway. The resulting centrifugal force within the capsule drives powder fluidization and emission from the capsule. The centrifugal force also leads to collisions between particles and with the capsule and raceway walls leading to dispersion of micronized drug from the carrier.

The entrained particles are subsequently drawn through a grid into the inhaler mouthpiece. The grid helps to align the airflow and leads to secondary dispersion of the dry powder by impaction of powder agglomerates with the grid. The grid further serves to reduce the probability that the capsule and capsule fragments generated during piercing from reaching the mouthpiece and being inhaled.

Entrained powder continues along the airflow path through an inlet funnel into the dispersion chamber. At the grid side, the inlet funnel is designed with a diameter that matches the diameter of the grid. At the dispersion chamber the inlet funnel is tapered to a small orifice, so as to enhance shear forces that powder agglomerates experience as they enter the dispersion chamber. The orifice diameter contributes critically to the resistance of the inhaler.

The dispersion chamber contains an actuator that is configured to oscillate within the dispersion chamber during inhalation. Powder dispersion within the dispersion chamber is enhanced by dynamic impaction between the actuator and the powdered medicament, and by turbulent forces generated within the dispersion chamber.

Powder exiting the dispersion chamber enters an outlet funnel, through which air and aerosolized medicament exit the inhaler and are delivered to the patient. The diameter of the outlet funnel at the exit from the dispersion chamber is designed to be comparable to the dispersion chamber diameter. The outlet funnel is tapered to maximize the area of the outlet from the device. This helps to reduce particle velocity and particle turbulence for the flow field of dispersed aerosol particles exiting the device. This in turn helps to limit particle deposition in the URT, helping to increase dose delivery to the lungs and small airways. The outlet funnel also contains a retaining member to prevent the actuator from being inhaled.

In another aspect, the dry powder inhaler may contain bypass airflow channels. The inclusion of bypass flow in the device enables reductions in device resistance and may increase sheath flow for powder exiting the device.

In a preferred embodiment, the dry powder inhaler has a high resistance to airflow (R = 0.14 to 0.25 cm $H_2O^{0.5}$ $L^{-1}$ min). Increasing device resistance increases dose delivery to the small airways. It may also lead patients to inhale with greater effort (i.e., increased pressure drops) without being instructed to do so. It has been shown that increasing device resistance may also decrease the incidence of post-inhalation cough for tussive APIs.

In another aspect described herein, a unit dose dry powder inhaler includes an inhaler base defining a capsule seat that is configured to hold a capsule containing a powdered medicament. The inhaler base includes at least one primary air intake that is in fluid communication with the capsule seat. The inhaler base may include at least one piercing member that is configured to pierce the capsule upon actuation. The primary air intake is configured to draw air into the capsule seat so as to generate rapid capsule precession and centrifugal forces that fluidize and disperse powder agglomerates. A grid is positioned between the inhaler base and a mouthpiece. Entrained powder is drawn through the grid resulting in dispersion of powder agglomerates via impaction forces with the static grid. The inhaler includes an inlet funnel positioned between the grid and a dispersion chamber. The inlet funnel tapers to a small inlet orifice into the dispersion chamber resulting in dispersion of powder agglomerates via shear forces generated at the orifice. The dispersion chamber is adapted to entrain powder from the inlet funnel. The dispersion chamber holds an actuator that is configured to oscillate within the dispersion chamber during inhalation resulting in dispersion of powder agglomerates by dynamic impaction with the actuator and increased turbulence within the dispersion chamber. The dispersion chamber may have a step increase in diameter relative to the small inlet orifice. The inhaler includes an outlet funnel through which air and aerosolized medicament exit the inhaler to be delivered to a patient. The outlet funnel is tapered to minimize particle velocity and turbulence at an exit of the mouthpiece to the patient.

In another aspect, a method (not according to the invention) for aerosolizing a powdered medicament to achieve improved delivery to the small airways is provided. The method includes providing an inhaler with four discrete dispersion mechanisms as described above (i.e., capsule motion, grid impaction, shear at inlet orifice, and dynamic impaction with the actuator and turbulent flow generated by the actuator in the dispersion chamber). The inhaler also contains an outlet funnel that minimizes particle velocity and turbulence for the powder flow field as it exits the inhaler. The method includes introducing air into the capsule seat via the at least one primary air intake to entrain powder within the air. The method may include generating centrifugal forces in the capsule seat to fluidize the powdered medicament prior to drawing the entrained powder through the grid. The method also includes drawing the entrained powder through the grid so as to impact at least a portion of the powdered medicament against the grid to disperse the at least a portion of the powdered medicament and dispersing powder agglomerates via increasing shear force produced by air flowing through the inlet funnel as the air approaches the dispersion chamber. The method further includes generating dynamic impaction and turbulent forces by inducing air to flow through the dispersion chamber to cause the actuator to oscillate within the dispersion chamber to disperse powder agglomerates and delivering the dispersed powder agglomerates to the patient's airway via the outlet funnel. The method may also include reducing the turbulent forces and reducing velocity of the deaggregated powder agglomerates in a flow field exiting the inhaler, thereby minimizing particle deposition in the patient's mouth and throat and ensuring a higher concentration of particles to peripheral regions of the patient's lungs.

BRIEF DESCRIPTION OF THE DRAWINGS

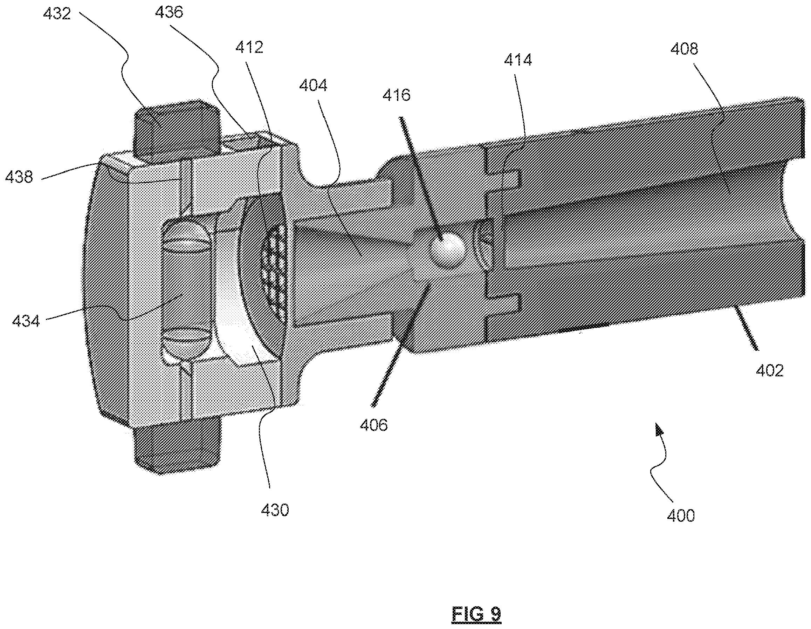

In the drawings, an embodiment of the invention is provided in Fig. 9 provided the diameters of the grid, of the inlet funnel at its grid side and of the orifice are according to claim 1. The rest of the embodiments described do not exhibit all the features of claim 1, but some of their features may be present in embodiments of the invention.

FIG. 1 depicts a cross-sectional view of a dry powder inhaler according to embodiments.

FIG. 2 depicts a cross-sectional view of a dry powder inhaler mouthpiece according to embodiments.

FIG. 3 depicts a top isometric view of the dry powder inhaler comprising the mouthpiece of FIG. 2.

FIG. 4 depicts a bottom isometric view of the dry powder inhaler mouthpiece of FIG. 2.

FIG. 5 depicts an exploded isometric view of a dry powder inhaler mouthpiece according to embodiments.

FIG. 6 depicts a bottom exploded isometric view of the dry powder inhaler mouthpiece of FIG. 5.

FIG. 7 depicts a cross-sectional view of the dry powder inhaler mouthpiece of FIG. 5 coupled with an inhaler base according to embodiments.

FIG. 8 depicts an inhaler base according to embodiments.

FIG. 9 depicts a cross-sectional view of a dry powder inhaler according to embodiments.

FIG. 10 depicts a cross-sectional view of an inhaler mouthpiece along with the critical dimensions of the dispersion chamber of FIG. 9 according to embodiments.

FIG. 11 depicts a dimensioned cross-sectional view of the inhaler body of FIG. 10.

FIG. 12 depicts a side cross-sectional view of the inhaler mouthpiece of FIG. 10.

FIG. 13 depicts a rear exploded view of the inhaler mouthpiece of FIG. 10 with a circular outlet.

FIG. 14 depicts a rear exploded view of the inhaler mouthpiece of FIG. 10 with an oval outlet.

FIG. 15 depicts a front exploded view of the inhaler mouthpiece of FIG. 10 with an oval outlet.

FIG. 16 depicts an isometric view of the inhaler mouthpiece of claim FIG. 10 with an oval outlet.

FIG. 17 is a flow chart depicting a process for delivering powdered medicament to a user's airway according to embodiments.

FIG. 18 is a chart depicting the aerodynamic particle size distributions of indacaterol inhalation powder in the RS01 DPI and prototype BP 2.8 SC DPI device tested at a 4 kPa pressure drop.

FIG. 19 is a plot of the fine particle distribution ($FPD_{S4-F}$) versus device resistance for a series of prototype DPI designs relative to the RS01.

FIG. 20 is a plot of impactor stage groupings (i.e., S3-F and S4-F) expressed as a percentage of the emitted dose for three vardenafil hydrochloride formulations administered with the RS01 DPI and three prototype DPIs.

FIG. 21 is a plot comparing the aerodynamic particle size distributions obtained for a 2.0% w/w vardenafil hydrochloride with 7.5% w/w lactose fines [Formulation HQ00006] administered with the RS01 DPI and a prototype dry powder inhaler with a 3.2 mm inlet orifice diameter and no bypass (NBP 3.2 SC).

FIG. 22 is a chart depicting variations in the $FPD_{S4-F}$ observed for a formulation comprising 2% w/w vardenafil hydrochloride as a function of variations in pressure drop (i.e., inspiratory effort) across the inhaler for the RS01 DPI and a prototype dry powder inhaler (NBP 3.2 SC).

DETAILED DESCRIPTION OF THE INVENTION

The ensuing description provides exemplary embodiments only, and is not intended to limit the scope, applicability or configuration of the invention, which is solely defined by the claims.

Rather, the ensuing description of the exemplary embodiments will provide those skilled in the art with an enabling description for implementing one or more exemplary embodiments. It being understood that various changes may be made in the function and arrangement of elements without departing from the scope of the invention as set forth in the appended claims.

For example, any detail discussed with regard to one embodiment may or may not be present in all contemplated versions of that embodiment. Likewise, any detail discussed with regard to one embodiment may or may not be present in all contemplated versions of other embodiments discussed herein. Finally, the absence of discussion of any detail with regard to embodiment herein shall be an implicit recognition that such detail may or may not be present in any version of any embodiment discussed herein. In all cases, the resulting subject-matter is an embodiment of the invention only if it falls within the scope of the claims.

Specific details are given in the following description to provide a thorough understanding of the embodiments. However, it will be understood by one of ordinary skill in the art that the embodiments may be practiced without these specific details, the resulting subject-matter being an embodiment of the invention only if it falls within the scope of the claims. For example, well-known processes, structures, and techniques may be not be discussed in detail in order to avoid obscuring the embodiments.

Similar devices and methods to those disclosed herein are disclosed in U.S. Patent Application Publication No. 2015/0246189 and U.S. Patent Application Publication No. 2015/0314086.

In a recent review Lavorini et al. (CHEST. 2017; 151:1345-1355) described the increased importance associated with improving delivery of aerosols to the small airways, viz: "During the past decade, there has been increasing evidence that the small airways (i.e., airways < 2 mm in internal diameter) contribute substantially to the pathophysiologic and clinical expression of asthma and COPD. Increasing the precision of drug deposition may improve targeting of specific diseases or receptor locations, decrease airway drug exposure and adverse events, and thereby increase the efficiency and effectiveness of drug delivery".

To achieve the desired improvements in deposition in the small airways, the inhaler designs described herein utilize four distinct powder dispersion elements: (a) shear forces resulting from rotation of the capsule in the capsule seat during inhalation; (b) static impaction forces resulting from impaction of particle agglomerates with a grid or screen during inhalation; (c) increased shear forces on the powder agglomerates at the orifice entry to the dispersion chamber; and (d) dynamic impaction and turbulent forces resulting from impaction of powder agglomerates with an axially oscillating actuator within the dispersion chamber.

The inhalers described herein may also enable improved delivery to arterioles in the pre-capillary portion of the pulmonary circuit. More specifically, dry powder inhalers described herein improve powder dispersion relative to current marketed capsule-based dry powders, which results in an increase in total lung dose, increased delivery to the peripheral regions of the lungs.

The inhalers described herein may also enable improvements in dose consistency relative to current marketed dry powder inhalers. This may be achieved by reducing deposition in the upper respiratory tract, and by decreasing the dependence of dose delivery on the inspiratory flow profile of the user.

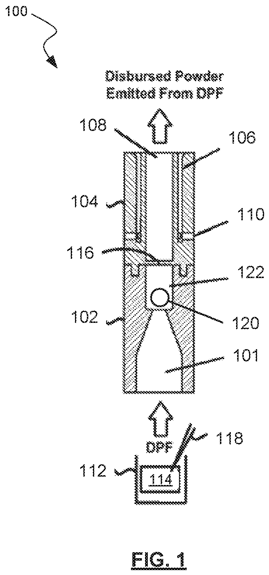

Referring now to **FIG. 1,** an example of a powder dispersion device or inhaler 100 is shown in accordance with the principles of the present disclosure. Inhaler 100 may include a first housing 102 comprising an inlet 101 (which may include a channel, lumen, conduit, funnel, and/or any other structure defining a flow path for a fluid) and a dispersion chamber 122. An actuator 120 may be positioned within the dispersion chamber 122. Inhaler 100 may further include a second housing 104 comprising one or more sheath flow channels 106 that surround and are not in fluid connection with a primary outlet 108 (which may include a channel, lumen, conduit, funnel, and/or any other structure defining a flow path for a fluid). In some embodiments, though not shown in FIG. 1, outlet 108 may be pitched or divergent from dispersion chamber 122 to the exit of inhaler 100. Thus, the diameter of the outlet 108 may increase linearly or non-linearly from dispersion chamber 122 to the exit of inhaler 100. This may assist in slowing powder flow and reducing turbulence in outlet 108 prior to delivery to the user. This may minimize impaction of the drug in the mouth and throat of the user.

In some embodiments, first housing 102 may be integrally formed with second housing 104. In one embodiment, dispersion chamber 122 and outlet 108 may have at least one common structural dimension, such as internal diameter for example. Additionally, second housing 104 may itself comprise of, be coupled to, or otherwise incorporated within, a mouthpiece adapted to be placed within the mouth of a patient, or in a nasal adapter adapted to conform to the nostrils of a patient. Inhaler 100 may further include a plurality of flow bypass inlets 110 that are formed within second housing 104. Flow bypass inlets 110 may be in fluid connection with sheath flow channels 106 as shown.

The inhaler 100 may further include a base 112, a retaining member 116 disposed at an end of the dispersion chamber opposite the inlet 101. A piercing member 118 may puncture or otherwise perforate a powder receptacle 114, such as a capsule, blister, or pod. In general, retaining member 116 may obstruct the opening or aperture, and be sized to permit air and powdered or otherwise aerosolized medicament to pass through retaining member 116, while preventing the possibility of actuator 120 from exiting dispersion chamber 122. The opening or aperture may, in some embodiments, be arranged and configured (e.g., diameter, pattern, etc.) to maintain desired fluid flow characteristics with inhaler 100, such that actuator 120 may disrupt and aerosolize medicament powder agglomerates within dispersion chamber 122 to provide for more effective deposition of medicament into the lungs of a patient.

In one example, a patient may prepare inhaler 100 by puncturing the capsule, blister, or transfer of a dose from a base 114, and then inhale, drawing air through dispersion chamber 122 which in turn draws the dry powder formulation (DPF) from the powder receptacle 114 into the adjacent dispersion chamber 122 via inlet 101, where actuator 120 is rapidly oscillating, creating high-energy forces that assist in increasing dispersion of the DPF in the airstream, thereby increasing drug delivery to the user. For example, oscillation of actuator 120 may strip drug from the surface of carrier particles in the DPF and/or de-agglomerate drug powder aggregates and drug-on-drug aggregates. Drug particles may then be deposited in lungs and airways of a patient from primary or outlet 108 based on direction of air flow through the device such as shown in FIG. 1. Such a design may be useful for effectively dispensing both traditional adhesive mixtures of micronized drug and coarse lactose monohydrate, and ternary blends comprising fine lactose particles and/or force control agents such as magnesium stearate. Also contemplated are pure drug-powder formulations where there are no carrier particles are present, and carrier-free engineered particles prepared by particle engineering techniques, including but not limited to spray-drying. Other embodiments having similar effects are possible within the scope of this disclosure.

In general, the resistance to flow of inhaler 100 may be adjusted by altering the geometry and/or arrangement of at least one of inlet 101, actuator 120, dispersion chamber 122, sheath flow channel 106, outlet 108, and flow bypass inlet(s) 110. Sheath flow channel 106 and flow bypass inlets 110 are in fluid communication and define a sheath flow path. Specifically, the length and diameter of dispersion chamber 122, as well as the diameter of actuator 120, and the ratio of the diameter of actuator 120 to the length and diameter of dispersion chamber 122 may all affect the flow resistance of inhaler 100. In particular, the size, number, and shape of bypass inlets 110 and the inlet channel at the junction with the dispersion chamber 122 have an effect on resistance of the inhaler 100. Additionally, flow bypass inlets 110 may be located radially around the body of second housing 104, and fluidly connected to sheath flow channel 106. In some embodiments however, the inhaler 100 may not include any flow bypass inlets. In one embodiment, flow bypass inlets 110 may include twelve individual channels located radially around the body of second housing 104. However, other embodiments are possible, as will be discussed below. For example, flow bypass inlets 110 may comprise of different numbers and diameters of individual channels and entry points into sheath flow channel 106. Further, one or more of flow bypass inlets 110 may be parallel through outlet 108, or may be in fluid connection with, and then diverge from, outlet 108. Other embodiments are also possible. It will be appreciated that, while not shown, bypass inlets such as those described above may be used in the inhaler 400 in some embodiments.

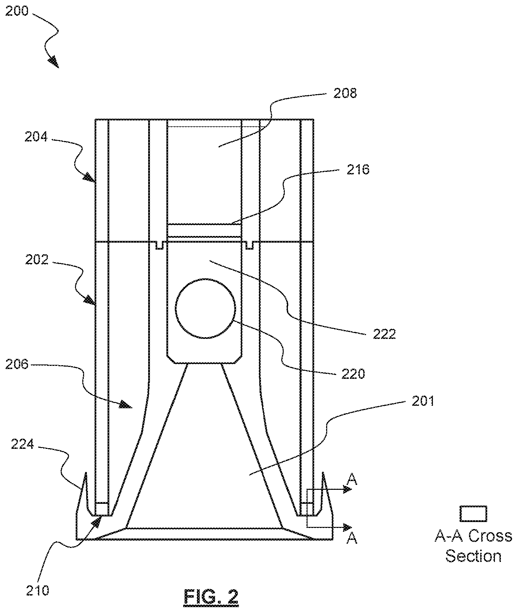

In an alternative construction to that shown in FIG. 1, **FIG. 2** shows another inhaler mouthpiece 200 having a first housing 202 and a second housing 204. As discussed above with respect to FIG. 1, first housing 202 and second housing 204 may be combined into a single piece housing in some embodiments, or be composed of more pieces in other embodiments.

Just as with FIG. 1, first housing 202 includes an inlet 201 (which may include a channel, lumen, conduit, funnel, and/or any other structure defining a flow path for a fluid), a dispersion chamber 222, and an actuator 220. However, in inhaler mouthpiece 200, first housing 202 also includes flow bypass inlets 210 which are in fluid communication with sheath flow channel 206 which begin in first housing 202 and continue through second housing 204 to the mouthpiece. This allows for sheath flow channel 206 to be longer than sheath flow channels 106 of FIG. 1 relative to the location of dispersion chambers 122, 222 and outlets 108, 208. Additionally, flow bypass inlets 210 may have a rectangular cross section in the A-A plane, as shown. Other shapes of bypass inlets may be possible in other embodiments, including square, polygon, circular, oval, or other shapes. Any other possible variations of inhaler 100 discussed above may also be present in inhaler 200.

First housing 202 may also include a skirt 224 which reduces the likelihood of foreign objects from entering and/or blocking flow bypass inlets 210. A second housing 204 may also include a retaining member 216 in order to retain actuator 220 in dispersion chamber 222, however the size and or shape may be changed from that shown in FIG. 1. While retaining member 116 of FIG. 1 may have constituted a cross-shaped member with each arm of the cross blocking dispersion chamber 122 at 90 degree intervals, a single crossbar may constitute retaining member 216 of FIG. 2, extending from one side of dispersion chamber 222 to the other. Other shapes of retaining member 216 are also possible in different embodiments.

The design of inhaler mouthpiece 200 may allow for the overall length of the combined first housing 202 and second housing 204 to be less than that of first housing 102 and second housing 104 from FIG. 1. For example, first housing 202 may have a length in the range from about 1 cm to about 3 cm. Second housing may have a length in the range from about 1 cm to about 2 cm. The modified size and shape of bypass inlets 210 may provide for lower air resistance through inhaler 200 compared to inhaler 100. This may assist some users during inhalation through inhaler 200, as well as have the consequent effect of improve carrying of DPF in the airstream of the outlet 208. At the same time, the overall length of inhaler 200 may be reduced, making it easier to store and transport.

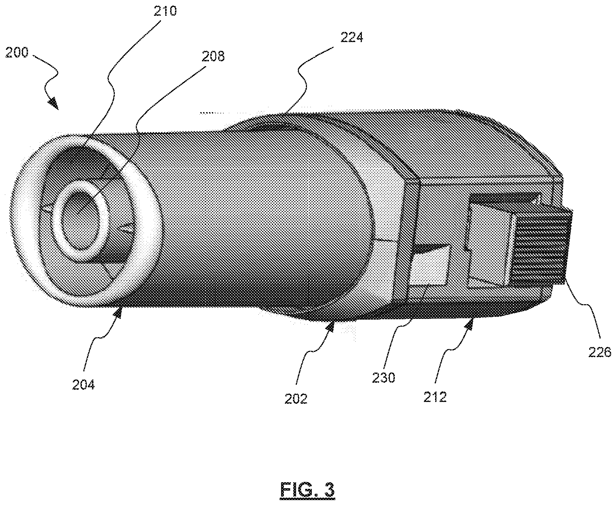

FIG. 3 shows a perspective view of inhaler mouthpiece 200, but also including the inhaler base 212 which includes one or more buttons 226 that each actuate a piercing member (not shown; within inhaler base 212) which may puncture or otherwise perforate a receptacle, such as a capsule (not shown; within inhaler base 212) as arranged or positioned within to distribute DPF or other provided substance into dispersion chamber 222. Also shown in FIG. 3 are outlet 208, flow bypass inlets 210, skirt 224, as well as primary air intake 230. Primary air intake 230 allows for air to carry DPF or any other substance from the inhaler base 212 through inlet 201 and into dispersion chamber 222 and onto the mouthpiece via outlet 208.

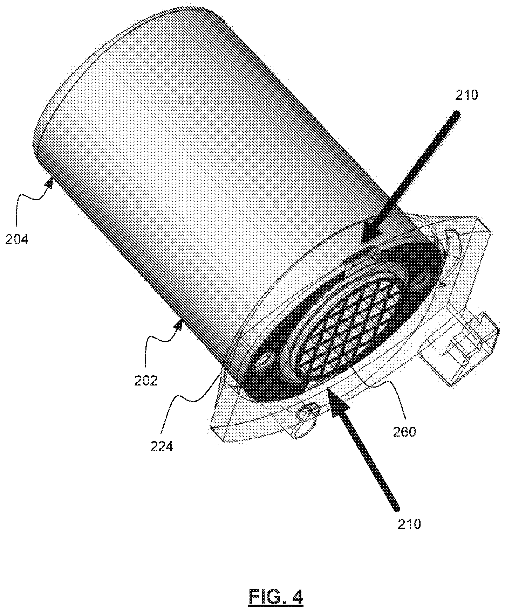

FIG. 4 shows a perspective view of first housing 202 and second housing 204 of the inhaler mouthpiece 200, separated from the inhaler base 212 of FIG. 3, and having skirt 224 shown only in wireframe perspective. The generally rectangular shape of flow bypass inlets 210 is shown in greater detail in this figure, although it will be appreciated that other sizes and/or shapes of bypass inlets may be used. As discussed above, flow bypass inlets 210 are normally shrouded by skirt 224, and are in communication with sheath flow channel 206 (not shown; within first housing 202 and second housing 204). While in this embodiment two flow bypass inlets 210 are present, in other embodiments only one flow bypass inlets may be present. Depending on the embodiment, there may be one, two, three, or more flow bypass inlets may be present. In some embodiments, the cross-sectional size of the rectangular flow bypass inlets 210 may be about 3.5 mm by about 1 mm. In other embodiments, other size flow bypass inlets 210 may be present. Additionally, shown in this figure is a screen or grid 260 which may separate dispersion chamber 222 of second housing 204 from the capsule seat 212, thereby containing actuator 220 within the dispersion chamber 222, and preventing the capsule or blister from entering dispersion chamber 222. Screen or grid 260 may also at least partially assist in dispersion of powder from the capsule, blister, or powder reservoir.

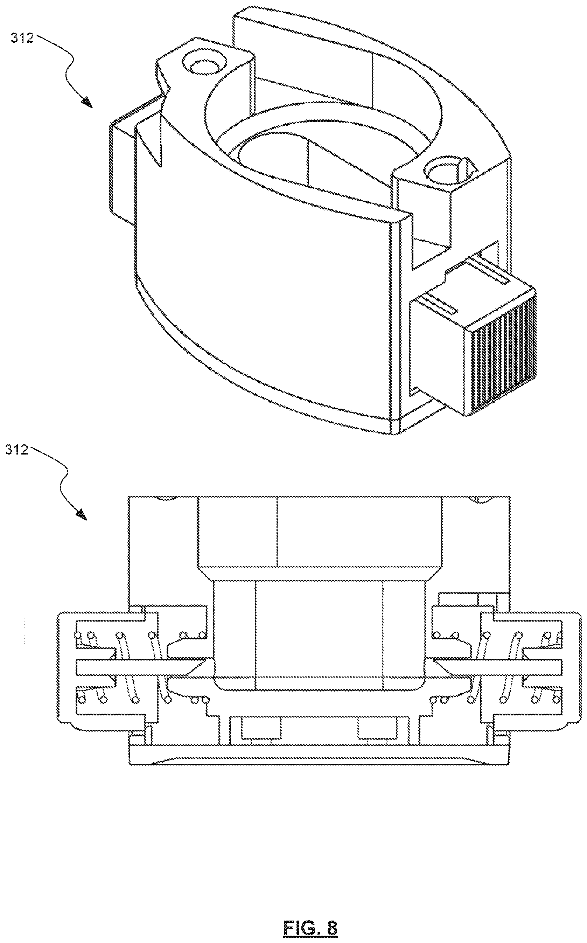

When coupled with an inhaler base, such as that shown in FIG. 8, the capsule-based inhaler mouthpiece 200 shown in FIG. 4 provides for four distinct powder dispersion elements in a single inhaler. This enables improved powder dispersion relative to current marketed capsule-based dry powders, which results in an increase in total lung dose, increased delivery to the peripheral regions of the lungs, and decreased dependence on the inspiratory flow profile of the subject. This represents the first capsule-based dry powder inhaler that incorporates four different dispersion elements operating by different mechanisms in a single device. Powder dispersion occurs via: (a) shear forces resulting from rotation of the capsule in the inhaler base 212 during inhalation; (b) static impaction forces resulting from impaction of particle agglomerates with the grid or screen 260 during inhalation; (c) increased shear forces on the powder agglomerates at the orifice entry to dispersion chamber 222; and (d) dynamic impaction and turbulent forces resulting from impaction of powder agglomerates with the axial oscillating actuator 220 in chamber.

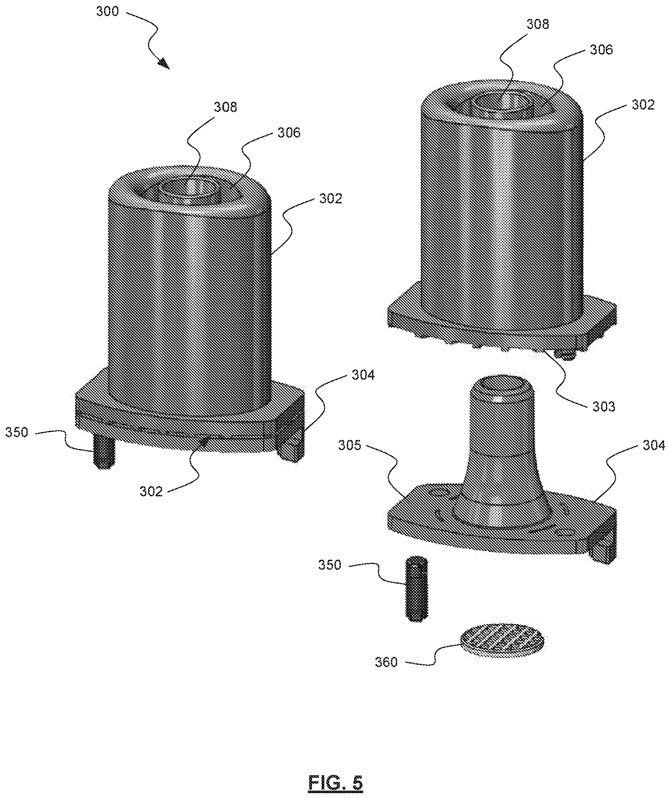

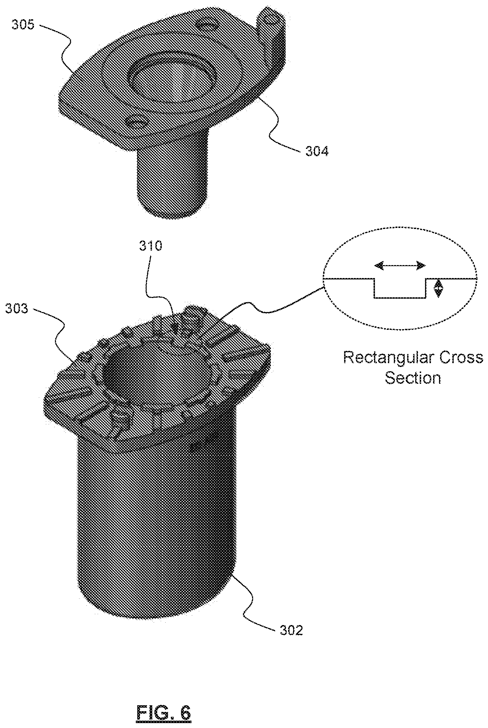

FIG. 5 shows an alternative embodiment from those presented above, though similar in function. Inhaler mouthpiece 300 includes a first housing 302 and a second housing 304. FIG. 6 shows first housing 302 and second housing 304 from an inverted angle.

In this embodiment, flow bypass inlets 310 are constructed by the interface created by a bottom 303 of first housing 302 meeting a top 305 of second housing 304. The shaping and sizing of bottom 303 and top 305 cause flow bypass inlets to be created by the assembly. Flow bypass inlets 310 are in fluid communication with sheath flow channels 306, which as in other embodiments, run parallel to outlet channel 308. Flow bypass inlets 310 may have a generally rectangular cross-sectional size of about 1 mm by about 0.5 mm. In this embodiment, eight flow bypass inlets 310 are present, but in other embodiments fewer or greater number of flow bypass inlets 310 may be present. Overall resistance through the device may be adjusted by changing the size and/or number of flow bypass inlets 310.

A chamber with oscillating actuator may be located within second housing 304 and provide fluid communication between the inhaler base (not shown) and the mouthpiece end of first housing 302, as in other embodiments disclosed herein. The dispersion chamber may then be in fluid communication with outlet channel 308, but separated therefrom by retaining member (e.g., cross, single cross-bar, etc.) as in other embodiments. In some embodiments, pins 350 couple first housing 302 with second housing 304, and possibly also the capsule seat. In these or other embodiments, a screen or grid 360 may also separate the dispersion chamber of second housing 304 from the inhaler base, thereby containing the actuator within the dispersion chamber. Additionally, screen or grid 360 may assist in straightening the air flow entering the dispersion chamber with oscillating actuator. The straightening of the airflow by the screen or grid 360 may also assist in consistent oscillation of the actuator in the dispersion chamber resulting in an enhanced or amplified sound produced by the oscillating actuator, which in turn provides audio assurance to the patient that they have inhaled sufficiently through the inhaler and that the device 300 is working as intended.

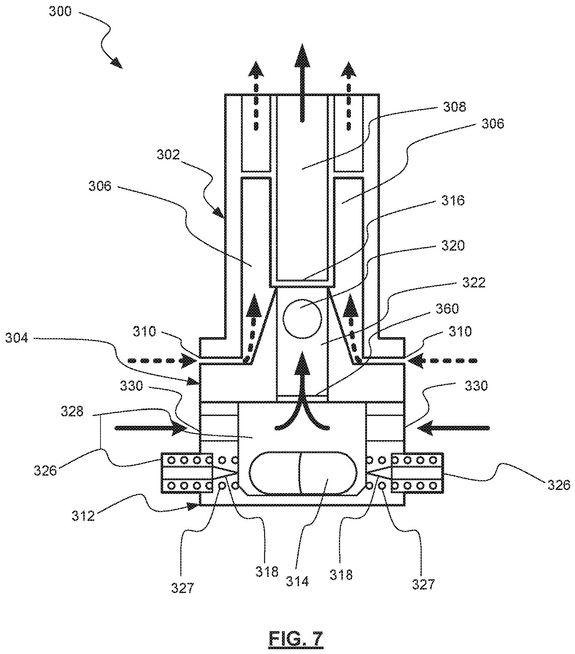

FIG. 7 shows a diagrammatic view (not to scale) of the embodiment of Figs. 4 and 5 in operation. Device 300 includes first housing 302 and second housing 304, as well as inhaler base 312. Inhaler base 312 may be the same as, or similar to, the Plastiape™ RS01 inhaler base available from Plastiape S.p.A. of Osnago, Italy, which is described in U.S. Patent Application No. 7,784,552.

When buttons 326 are actuated inward, overcoming compression springs 327, piercing members 318 puncture the capsule, and DPF or another substance is released into the airstream from the capsule 314, as shown by the solid arrows which is produced by a user pulling air through the device from its entry point at primary air intakes 330. For example, airflow through the inhaler base 312 lifts the capsule and results in precession at high speed around its main axis within a raceway 328 of the inhaler base 312. A critical feature of the invention is the resulting centrifugal force within the capsule that drives powder fluidization and emission from the capsule. The centrifugal force also leads to collisions between particles and with the capsule and raceway walls leading to dispersion of micronized drug from the carrier. The fluidized DPF is then pulled past screen or grid 360, through dispersion chamber 322 and oscillating actuator 320, past retaining member 316, and out through outlet 308 (which may include a channel, lumen, conduit, funnel, and/or any other structure defining a flow path for a fluid). Meanwhile, air is also being drawn by the user through flow bypass inlets 310 and through sheath flow channels 306.

FIG. 8 shows a more detailed cut-away view of inhaler base 312, as well as a perspective view thereof. The shape of the capsule seat within the inhaler base 312 may compliment the shape of the capsule 314. In the embodiment shown, the capsule seat is shaped and configured to receive a complimentary shaped capsule of DPF. In some embodiments, the capsule may spin as described above once punctured and air is drawn through the device.

In some embodiments, all of the patient's inhaled air passed directly though the dispersion chamber containing the oscillating actuator. In some optional embodiments, some air may pass through bypass channels. For example, in some embodiments about 10% to about 90%, about 20% to about 80%, about 30% to about 70%, about 40% to about 60% of the total air flow passing through the device proceeds through the flow bypass inlets and sheath flow channel. In some of these embodiments, about 50% of the total air flow passing through the device proceeds through the flow bypass inlets and sheath flow channel. In some embodiments, lower percentage ranges of flow through the flow bypass inlets and sheath flow channel may assist in increasing the air flow through the outlet channel, thereby increasing the effectiveness of the dispersion mechanisms discussed above. In other embodiments, more or less of the total air flow passing through the device may proceed through the flow bypass inlets and sheath flow channel. The remaining amount of air flow passes through the capsule seat, central chamber with actuator, and outlet channel.

Referring now to **FIG. 9,** an example powder dispersion device or inhaler 400 is shown in accordance with an embodiment of the invention. The inhaler 400 includes an inhaler base 428 defining a capsule seat 430 that may be configured to hold a capsule 434 containing a powdered medicament. The capsule 434 is pierced on either end by depressing two spring loaded buttons 432 that each bring forward a metal piercing needle 438. The needles 438 pierce a single hole on either side of the capsule 434. The base 428 also defines at least one primary airflow inlet 436. Air drawn in through the primary airflow inlet 436 flows through the capsule seat 430 and lifts the capsule 434, causing precession of the capsule 434 high speed around its main axis. The resulting centrifugal force within the capsule 434 drives powder fluidization and emission of the powdered medicament from the capsule 434. The centrifugal force also leads to collisions between particles and with the capsule and raceway walls leading to dispersion of micronized drug from the carrier.

The entrained particles are subsequently drawn through a grid 412 into the inhaler mouthpiece. Grid 412 provides static impaction forces that result from impaction of particle agglomerates with the grid or screen 412 as air and powdered medicament flow through the inhaler 400 during inhalation. For example, the entrained particles from the capsule seat are drawn through the grid 412 prior to entering an inlet funnel 404, with the grid 412 helping to align the airflow and leading to secondary dispersion of the dry powder by impaction of powder agglomerates with the grid 412. The grid 412 further serves to remove any fragments of capsule generated during piercing, while further preventing the capsule from reaching the mouthpiece and being inhaled. Grid 412 may include one or more cross-members that may be arranged in a symmetrical or non-symmetrical manner along one or more axes. Cross-members may be straight and/or arcuate. The diameter of the grid 412 may be determined by the external diameter of the inhaler 400, but typically has a diameter of between about 10 mm and 20 mm, oftentimes about 14 mm.

Entrained powder continues along the airflow path through an inlet funnel 404 into a dispersion chamber 406. At the grid side, the inlet funnel 404 has a diameter that matches that of the grid 412 to increase the amount of static impaction of the powdered medicament. The grid side of the inlet funnel 404 has a diameter matching the diameter of a 10 mm to 20 mm grid 412. As it extends to the dispersion chamber 406, the inlet funnel 404 tapers, in a linear and/or non-linear manner, to a small orifice 410, so as to enhance shear forces that powder agglomerates experience as they enter the dispersion chamber 406. The orifice diameter contributes critically to the resistance of the inhaler. The diameter of the orifice 410 is from 2.8 to 3.4 mm.

As the diameter of the grid side of the inlet funnel 404 is increased for inhaler designs that utilize bypass channels, the aerosol performance decreases. This is not surprising, as one would expect the shear forces acting on the particles would decrease with increased diameters. However, it was surprisingly discovered that this is not true with inhaler designs that include no bypass channels (all airflow through the inhaler 400 flows through the inlet funnel 404, dispersion chamber 406, and outlet funnel 408). In fact, aerosol performance increased as the diameter of the grid side of the inlet funnel 404 increased, reaching a maximum in aerosol performance at a diameter of about 3.2 mm, after which performance declines.

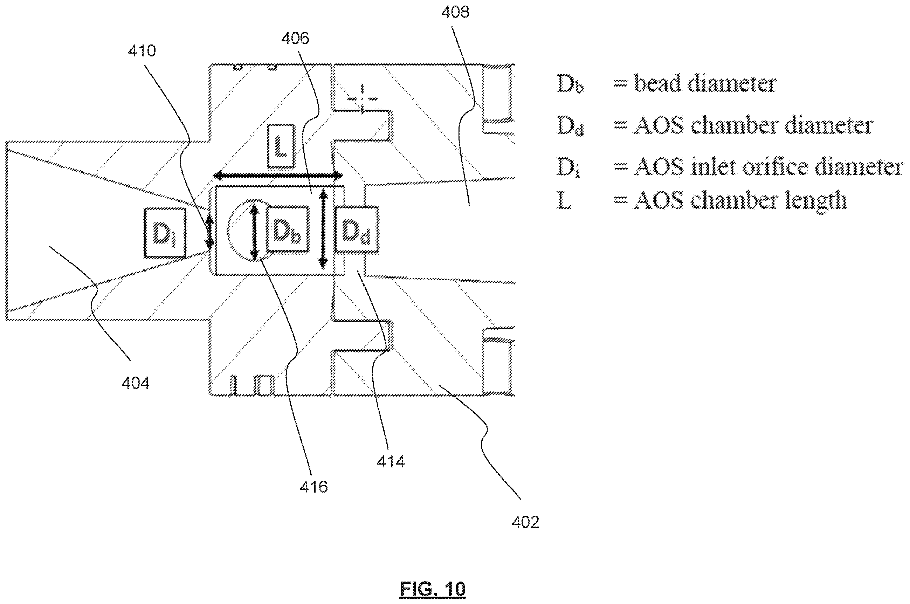

The dispersion chamber 406 contains an actuator 416, such as a bead or sphere, that is configured to oscillate within the dispersion chamber 406 during inhalation. Powder dispersion within the dispersion chamber 406 is enhanced by dynamic impaction between the actuator 416 and the powdered medicament, and by turbulent forces generated within the dispersion chamber 406. The dispersion chamber 406 may define a straight flow path such that actuator 416 may oscillate, generally linearly in certain embodiments, along an axis of the dispersion chamber 406 when the patient inhales through the inhaler 400. As shown schematically in **FIG. 10,** in order to optimize the oscillation of the actuator 416 a ratio of the diameter ($D_i$) of the inlet funnel 404 at the connection with the dispersion chamber 406 with the diameter ($D_d$) of the dispersion chamber 406 is between about 0.4 and 0.6, with optimal oscillation of the actuator 416 occurring at a higher end of the range. A ratio of the length (L) of the dispersion chamber 406 to the diameter ($D_b$) of the actuator 116 is between about 2.0 and 3.5. As just one example, the diameter ($D_d$) of the dispersion chamber 406 may be about 5.9 mm, while the length (L) of the dispersion chamber 406 is about 10 mm. The diameter ($D_i$) of the inlet funnel 404 at the connection with the dispersion chamber 406 is between about 2.8 and 3.4 mm, and the diameter ($D_b$) of the actuator 116 is about 4 mm.

In some embodiments, the dispersion chamber 406 may be designed such that a sudden flow stream expansion may occur when the relatively "small" cross-sectional orifice 410 opens abruptly into a larger cross-sectional fluid flow path of or defined by the dispersion chamber 406 shown as the step increase at the junction between the orifice 410 and the dispersion chamber 406. For example, a diameter of the orifice 110 of between 2.8 mm and 3.4 mm may have a step increase to a diameter of the dispersion chamber 406 of between about 4 and 12 mm, preferably between about 5 and 8 mm. In such embodiments, high-energy forces may develop by within the dispersion chamber 406. In one aspect, this may be due to relatively "low" pressure regions induced by relatively "high" velocity fluid entering the dispersion chamber 406, where a portion of the flow stream detaches. Other mechanisms may contribute to the development of high-energy fluid flow within the dispersion chamber 406 as well. Further, such high-energy fluid flow, along with mechanical impact forces, may disrupt and aerosolize medicament powder agglomerates within the dispersion chamber 406 to provide for more effective deposition of medicament into the lungs of a patient.

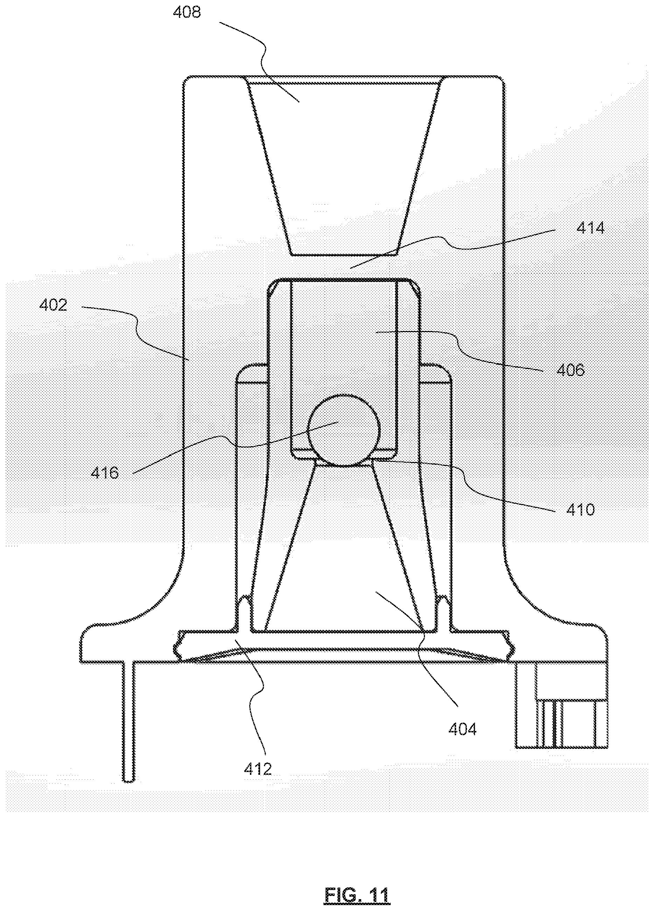

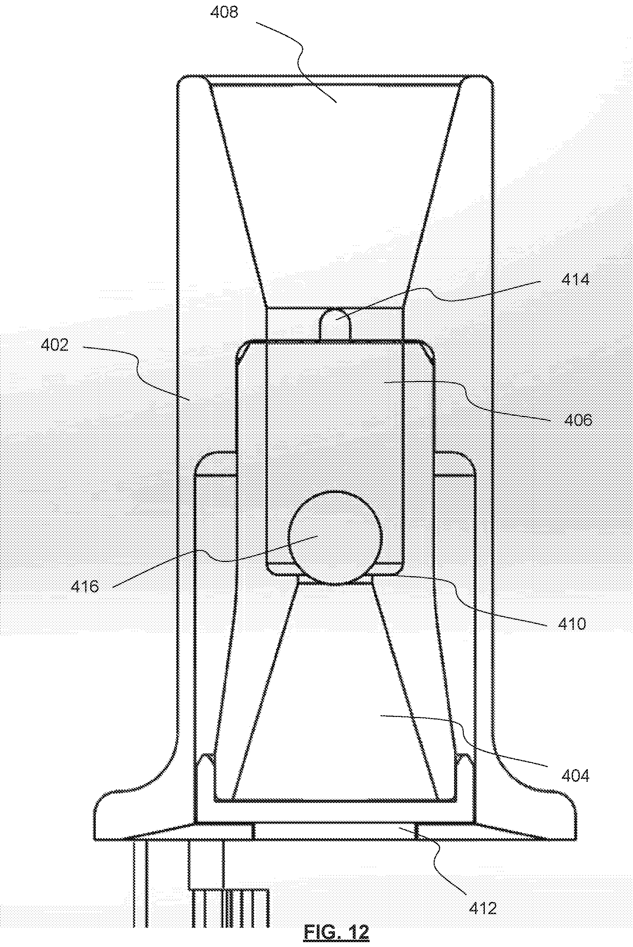

Returning to FIG. 9, a retaining member 414 may be positioned at an exit end of the dispersion chamber 406. Retaining member 414 may be sized to permit air and powdered or otherwise aerosolized medicament to pass through retaining member 414, while preventing the possibility of the actuator 416 from exiting the dispersion chamber 406. Retaining member may include one or more bars extending across the opening at an exit end of the dispersion chamber 406. For example, as shown in FIGs **11 and 12** a single bar extends across the opening at the exit end of the dispersion chamber 406. It will be appreciated that other numbers and/or shapes of retaining members 414 may be used to prevent the actuator 416 from exiting the dispersion chamber 406. The selection of a number, size, shape, and/or other arrangement of retaining members 414 may be based on the desired flow characteristics, the relative sizes of the actuator 416 and the opening between the dispersion chamber 406 and the outlet funnel 408, and/or other factors. The opening at the exit end of the dispersion chamber 406 may be arranged and configured (e.g., diameter, pattern, etc.) to maintain desired fluid flow characteristics with inhaler 400, such that actuator 416 may disrupt and aerosolize medicament powder agglomerates within the dispersion chamber 406 to provide for more effective deposition of medicament into the lungs of a user.

Powder exiting the dispersion chamber 406 enters an outlet funnel 408, through which air and aerosolized medicament exit the inhaler 400 and are delivered to the patient. The diameter of the outlet funnel 106 at exit from the dispersion chamber 406 is designed to be comparable to the dispersion chamber 406 diameter. The outlet funnel 408 tapers outward, linearly and/or non-linearly, to maximize the area of the outlet of the inhaler 400. This helps to reduce particle velocity and particle turbulence for the flow field of dispersed aerosol particles exiting the device. This in turn helps to limit particle deposition in the URT, helping to increase dose delivery to the lungs and small airways. The outlet funnel 408 also contains a retaining member to prevent the actuator from being inhaled.

Dimensions at the outlet side of the outlet funnel 408 may be optimized to achieve maximal surface area. In some embodiments, the outlet funnel 408 may have a conical frustum shape with a circular cross-section, while in other embodiments, a conical frustum having an elliptical cross-section may be used. Other shapes may be contemplated in accordance with the present invention.

For example, a diameter of the exit side of the outlet funnel 408 may be between about 8 mm and 14 mm for a circular no bypass design, such as about 11 mm. For an elliptical (oval) outlet, the short dimension is the same as is described above for the circular outlet, and the long axis is about 1.5-fold greater (i.e., 12 mm to 21 mm). Particularly preferred is an oval outlet with dimensions of 16 mm x 11 mm. For bypass designs, the outlet diameter is decreased, to between 6 mm and 12 mm, such as about 10 mm.

Inhaler 400 includes no bypass inlets or sheath flow channels that would create airflow through the inhaler 400 in a location that is separate or isolated from the flow path defined by the inlet funnel 404, dispersion chamber 406, and outlet funnel 408. Such designs help increase the flow resistance of the inhaler design. No bypass designs are particularly preferred because the higher resistance devices lead to improved small airway delivery. They also may help patients who provide low effort on inhalation to inhale with more effort. Patients achieve their highest flow rate when inhaling against no resistance, and their highest pressure drop (i.e., greatest effort) when inhaling against infinite resistance. Finally, inhaling at lower flow rates with higher resistance devices may help to reduce the incidence of post-inhalation cough for tussive APIs. Such benefits are generally realized at a resistance of between about 0.14 and 0.25 cm $H_2O^{0.5}$ $L^{-1}$ min, and preferably between 0.16 and 0.19 cm $H_2O^{0.5}$ $L^{-1}$ min.

In general, the resistance to flow of the inhaler may be adjusted by altering the geometry and/or arrangement of the inlet funnel 404, the actuator 416, the dispersion chamber 406, and/or the outlet funnel 408. Specifically, the length and diameter of dispersion chamber 406, as well as the diameter of actuator 416, and the ratio of the diameter of actuator 416 to the length and diameter of dispersion chamber 406 may all affect the flow resistance of the inhaler as discussed in greater detail in relation to FIG. 10 above. However, the resulting inhaler is according to the invention only if it falls within the scope of claim 1.

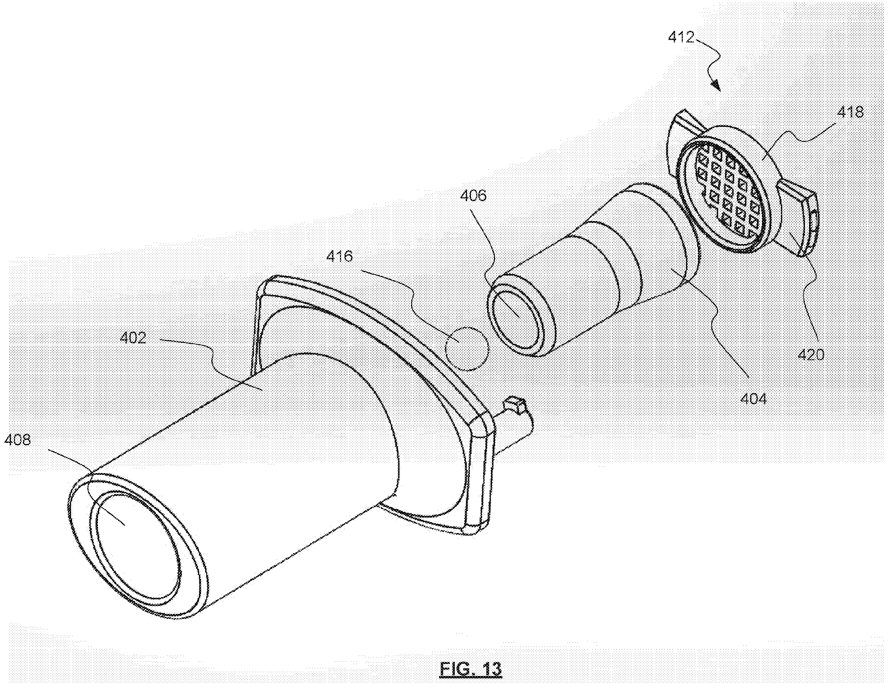

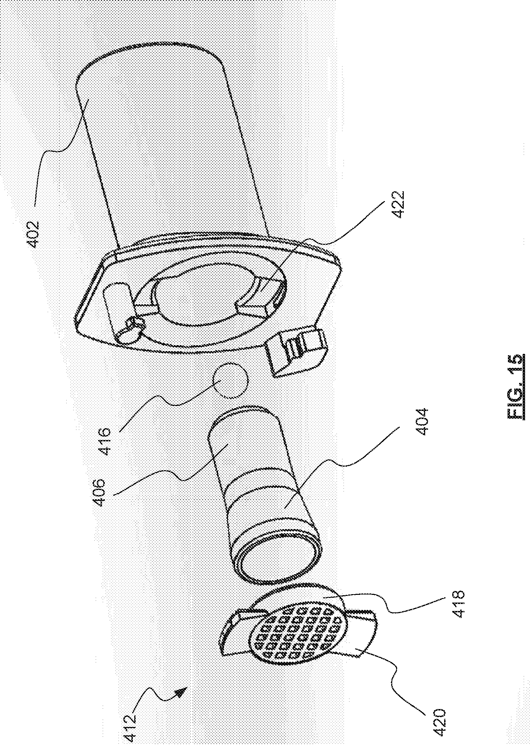

It will be appreciated that inhaler mouthpiece 402 and/or the components provided therein may be formed as a single piece or may be formed of multiple components that are permanently and/or detachably coupled with one another. For example, as shown in **FIGs. 13-15,** the inhaler mouthpiece 402 includes the outlet funnel 408, with the inlet funnel 404 and dispersion chamber 406 forming a separate component. Actuator 416 may be configured to be inserted within the dispersion chamber 406, such as from a downstream direction. Retaining member 414 may be attached to a downstream end of the dispersion chamber 406 and/or may be a part of the inhaler body 402 such that as the component forming the inlet funnel 404 and the dispersion chamber 406 is inserted into the inhaler mouthpiece 402, the downstream end of the dispersion chamber 406 contacts and couples with the retaining member to prevent the actuator 416 from moving out of the dispersion chamber 406. Grid 412 may be configured to interface with an upstream end of the component that includes the inlet funnel 404 and the dispersion chamber 406. For example, the grid 412 may be configured to receive the upstream end of the component such that a rim 418 of the grid 412 encircles all or a portion of the end of the component and/or an upstream end of the inlet funnel 404. As best seen in **FIG. 15,** grid 412 may include one or more flanges 420 that may extend outward from the rim 418. In some embodiments, the flanges 420 may extend around an entire periphery of the rim 418, while in other embodiments the flanges 420 extend from only a portion of the rim 418. Flanges 420 may be used to assist in aligning and seating the grid 412 onto the inhaler mouthpiece 402. For example, the inhaler mouthpiece 402 may define a countersink and/or counter bore or similar receptacle 422 that is sized and shaped to match the one or more flanges 420. The flanges 420 may be inserted into the receptacle 422 to set a relative position and depth of the grid 412 relative to the inhaler body 402 and inlet funnel 404. The various components of the assembly that forms inhaler 400 may be coupled to one another using a "snap-fit" or a "pressure-fit" mechanism.

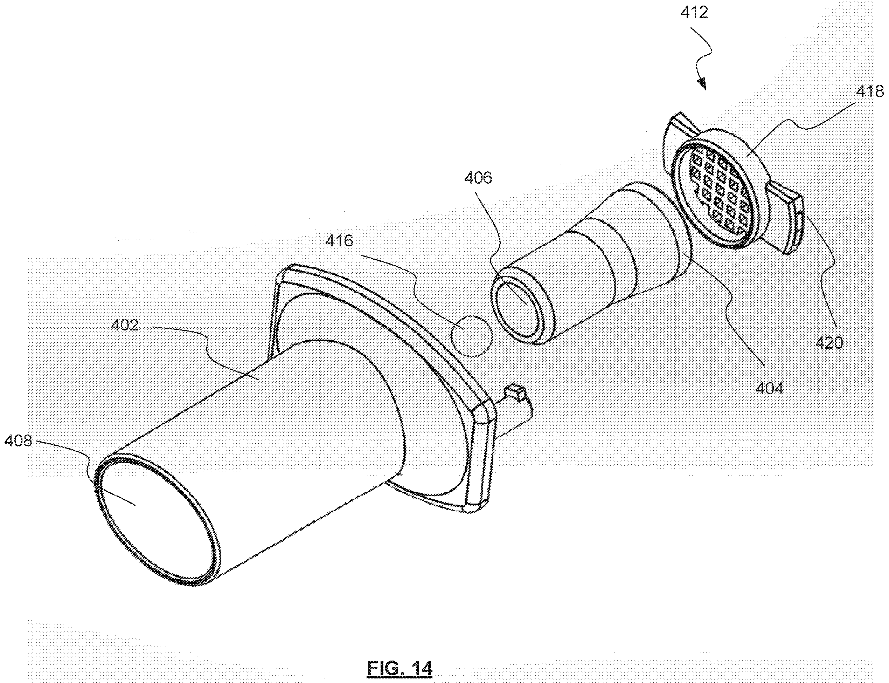

**FIGs. 13 and 14** demonstrate that embodiments with a circular conical frustum outlet funnel 408 (FIG. 13) and embodiments with an elliptic conical frustum outlet funnel 408 (FIG. 14) may be formed and assembled in a similar manner. While shown here having an exterior of inhaler mouthpiece 402 being elliptical at the downstream side, it will be appreciated that other shapes may be possible. Additionally, while FIG. 14 shows the outlet funnel 408 and exterior of inhaler mouthpiece 402 to have similar elliptical shapes, it will be appreciated that the ratio of height and width of the elliptical shape of the outlet funnel 408 may be different than the ratio of height and width of the elliptical shape of the exterior of the inhaler body 402. Embodiments with elliptical outlet funnels 408 that have shapes matching the exterior of the inhaler mouthpiece 402 maximize the size of the exit of the mouthpiece of the inhaler 400, thereby maximizing the slowing of aerosol and the reduction of mouth deposition of medicament particles.

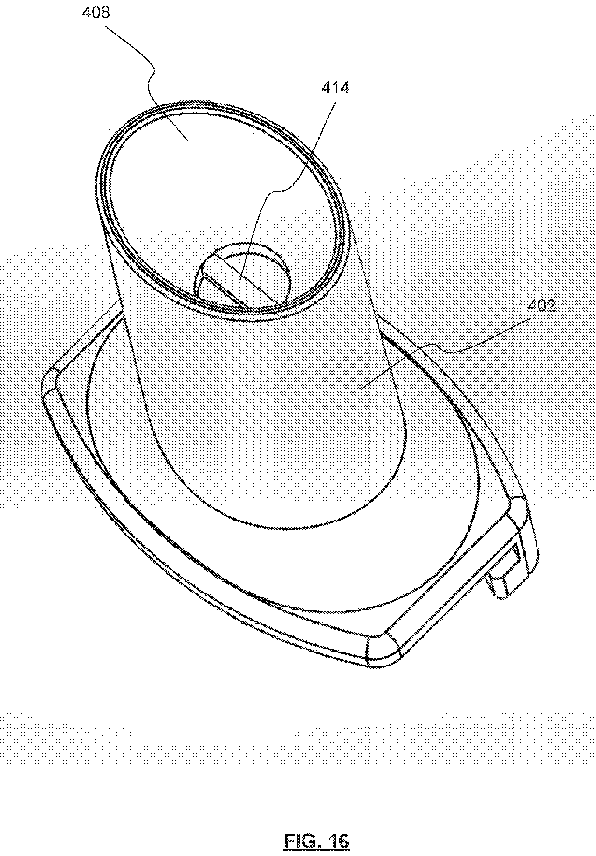

**FIG. 16** depicts an isometric view of inhaler mouthpiece 402. Here, retaining member 414 is shown positioned between the dispersion chamber 406 and the outlet funnel 408. As discussed above, the retaining member 414 may be a single straight bar of material as depicted here, or may include multiple bars that each have the same and or different shape and/or orientation. As depicted here, retaining member 414 is formed as part of the inhaler mouthpiece 402 such that once the dispersion chamber 406 is inserted within the inhaler mouthpiece 402, the retaining member 414 engages with a downstream end of the dispersion chamber 406 to prevent the actuator 416 from exiting the dispersion chamber 406. Outlet funnel 408 may taper outward from a smaller opening near the retaining member 414 to a wider opening near the exit of the inhaler body 402. This helps to reduce the turbulence and velocity of particles in the flow field exiting the inhaler 400, thereby minimizing particle deposition in the mouth and throat and ensuring a higher concentration of the particles is delivered to the peripheral regions of the lungs.

In one example, a patient may prepare the inhaler 400 by puncturing a capsule 434 that is placed within a capsule seat 430 that is coupled with the inhaler mouthpiece 402. The patient may then inhale, drawing air through the inlet funnel 404, dispersion chamber 406, and the outlet funnel 408, which in turn draws the dry powder formulation (DPF) from the capsule seat 430 into the dispersion chamber 406 via the inlet funnel 404, where actuator 416 is oscillating, which creates high-energy forces that assist in increasing dispersion of the DPF in the airstream, thereby increasing drug delivery to the user. The increased dispersion results from the actuator 416 stripping drug from the surface of carrier particles in the DPF and/or de-agglomerate drug powder aggregates and drug-on-drug aggregates while the actuator 416 is oscillating within the dispersion chamber 406. Drug particles may then be deposited in lungs and airways of a patient from the outlet funnel 408.

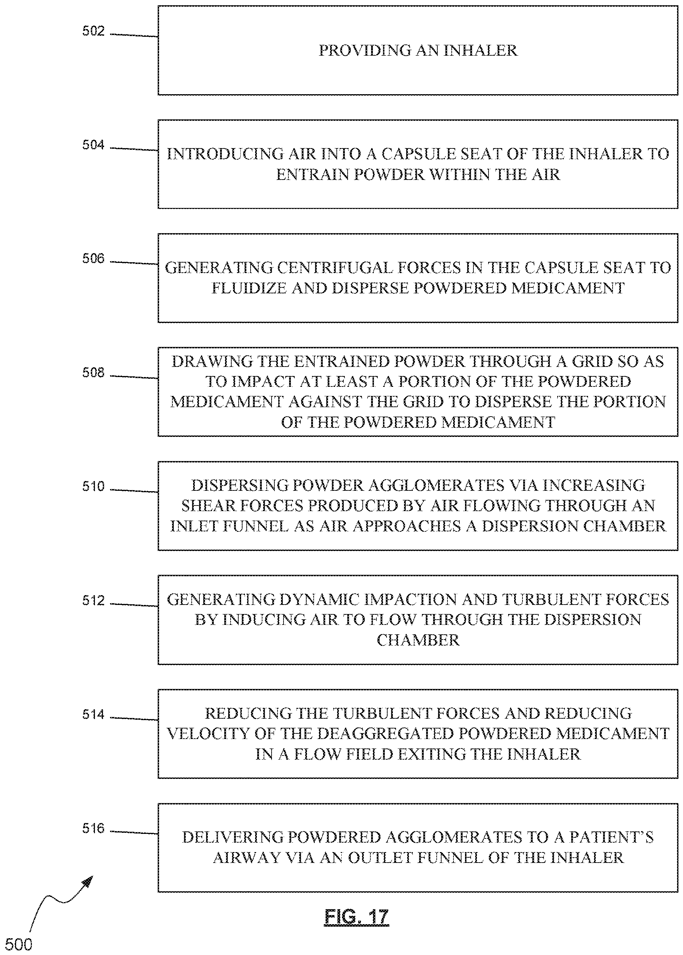

FIG. 17 depicts a process 500 for delivering aerosolized medicament to peripheral regions of a user's airways. In some embodiments, the aerosolized powdered medicament comprises particles with an impaction parameter that enables efficient delivery to patient airways of less than about 2 mm in internal diameter. Process 500 may begin at block 502 by providing an inhaler, such as any of the inhalers described herein. For example, the inhaler may include a base defining a capsule seat and a primary air intake, a grid disposed between the base and an inlet funnel, a dispersion chamber holding an actuator that is configured to oscillate within the dispersion chamber during inhalation, and an outlet funnel through which air and aerosolized medicament exit the inhaler to be delivered to a patient. Air is introduced into the capsule seat via the primary air intake to entrain powder within the air at block 504. In some embodiments, centrifugal forces are generated in a capsule seat of the inhaler to fluidize and disperse the powdered medicament at block 506. This may be done using a capsule seat having primary air intakes that are configured to draw in air in a swirling motion that may serve to rotate a capsule or other powder receptacle. At block 508, the process 500 may include drawing the entrained powder through the grid so as to impact at least a portion of the powdered medicament against the grid to disperse the at least a portion of the powdered medicament. Powder agglomerates may be dispersed via increasing shear forces produced by air flowing through the inlet funnel as the air approaches the dispersion chamber at block 510.

At block 512 dynamic impaction and turbulent forces are generated by inducing air to flow through the dispersion chamber to cause the actuator to oscillate within the dispersion chamber to deaggregate the powdered medicament within the dispersion chamber to be aerosolized and entrained by the air and delivered to the patient through the outlet channel. Process 500 may also include reducing the turbulent forces and reducing velocity of the deaggregated powdered medicament in a flow field exiting the inhaler at block 514, thereby minimizing particle deposition in a user's mouth and throat and ensuring a higher concentration of particles to peripheral regions of the user's lungs. The dispersed powder agglomerates may be delivered to the patient's airway via the outlet funnel at block 516. In some embodiments, all airflow introduced into the inhaler flows through the outlet channel, while in other embodiments, a portion of the airflow may flow through bypass inlets and/or a sheath channel.

EXAMPLES

**Example 1. Impact of dry powder inhaler design on the aerodynamic particle size distribution of Arcapta®
(indacaterol inhalation powder)**

Two independent studies were conducted to assess the impact of various dry powder inhaler designs on aerosol performance of the commercial Arcapta® Neohaler® (indacaterol inhalation powder) drug product. Indacaterol is a long-acting beta-agonist, indicated for the treatment of patients with chronic obstructive pulmonary disease. The Arcapta formulation is comprised of an adhesive mixture of 75 $\mu$g of micronized indacaterol maleate blended with 25 mg of lactose monohydrate carrier particles. The formulated powder is encapsulated in size 3 hard gelatin capsules and administered to patients with the Neohaler dry powder inhaler. The Neohaler is equivalent to an RS01 dry powder inhaler (described below) in terms of its aerosol engine, differing only in its external appearance and usability characteristics.

The RS01 DPI is a portable, manually-operated, breath-activated, unit-dose, capsule-based dry powder inhaler of medium resistance (R = 0.10 cm $H_2O^{0.5}$ $L^{-1}$ min). It is intended for administration of active pharmaceutical ingredients to the lungs via oral inhalation. The device uses no batteries or electronics.

Key elements of the RS01 device include: a base unit wherein the capsule is loaded and prepared for inhalation. The capsule is pierced on either end by depressing two spring loaded buttons that bring forward metal piercing pins.

The base also contains holes for the main airflow through the device. Airflow through the capsule seat lifts the capsule and results in precession at high speed around its main axis. The resulting centrifugal force within the capsule results in fluidization of the powder and emission from the capsule. The centrifugal force also leads to collisions between particles and with the capsule wall leading to dispersion of micronized drug from the carrier. The entrained particles are drawn through a grid that helps to align the airflow and leads to secondary dispersion of the dry powder by impaction of powder agglomerates with the grid. The grid further serves to remove any fragments of capsule generated during piercing, while further preventing the capsule from reaching the mouthpiece and being inhaled.

The long mouthpiece on the RS01 extends like a chimney from the base. The extended mouthpiece ensures that the velocity of particles exiting the device are consistent with predictions of particle velocity based on mouthpiece diameter and flow rate. The flow-field exiting the device has a low velocity and minimal turbulence, helping to minimize particle deposition in the mouth. When inserted into the mouth, the long mouthpiece also acts as a 'tongue depressor', further ensuring that particles exiting the mouthpiece do not impact with the tongue and teeth.

In this example, an axial oscillating sphere (AOS™) dry powder inhaler mouthpiece design, as described in the present disclosure, are coupled with the low resistance RS01 base unit. The inhaler 'engine' of the present invention is introduced within the mouthpiece of the RS01 design. For the designs studied herein, the external dimensions of the mouthpiece are the same as the RS01. This facilitates the use of the same high-speed manufacturing lines during device assembly. The nomenclature used to describe the prototype inhaler devices is as follows. The initial letters refer to whether the device contains a bypass feature (BP), or whether there is no bypass (NBP) in the design. This is followed by the diameter of the inlet orifice to the dispersion chamber, i.e., 2.8, 3.2, or 3.4 mm. Finally, there are two letters, which stipulate the nature of the retaining member (i.e, a single bar (S) or a cross (C)), and the shape of the airflow path at the mouthpiece exit (i.e., spherical (S) or oval (O)). Thus, a NBP 3.2 SO refers to a no bypass design with a 3.2 mm inlet orifice, a single bar retaining member, and an oval mouthpiece exit. Remaining design features of the devices that are held constant are detailed in the captions to the tables.

Aerodynamic particle size distributions (APSD) were determined with a Next Generation Impactor (NGI). The NGI separates particles onto various stages based on their inertial impaction parameter. When comparing across devices of differing resistance, it is imperative that they be compared at a constant pressure drop (i.e., a constant level of patient effort), as opposed to a constant flow rate. Accordingly, one Arcapta capsule was actuated per APSD determination. Each capsule was actuated at a 4 kPa pressure drop, for a total inhaled volume of 4 L per capsule. The impactor stages were coated with a 50% v/v Tween 20 solution in methanol to prevent re-entrainment of particles. Prior to testing, each dry powder inhaler was primed by actuating two doses to waste prior to initiation of testing. Drug depositing on individual impactor stages (1 through MOC of Filter (F))) were extracted with 10 mL of a diluent comprising equal volumes of methanol and water. The induction port and pre-separator were extracted with 50 mL of diluent. The mass of indacaterol in the test samples was quantitated via high performance liquid chromatography (HPLC) according to a method described previously (Weers et al: J Aerosol Med Pulm Drug Deliv. 2015; 28:268-280).

In the first study **(Table 1),** the aerosol performance of different dry powder inhaler designs as described herein were compared with results for the RS01 dry powder inhaler. The prototype inhaler designs contain the dispersion elements present in the RS01 device (i.e., the rotating capsule and grid), while adding two additional dispersion elements that differ mechanistically, i.e., shear forces generated by the small inlet orifice entering the dispersion chamber, and the dynamic impaction forces created with the axial oscillating sphere. Due to the inclusion of the inhaler engine in the mouthpiece, the prototype designs have significantly higher device resistances as compared to RS01. Hence, for a given pressure drop, the prototype designs will be actuated at lower flow rates. Device resistance in the prototype designs is varied via changes in the inlet diameter to the dispersion chamber and/or by inclusion of a bypass circuit.

The impactor measured emitted dose (ED) was highest for the RS01 followed by the AOS designs containing BP, and then the prototype designs with NBP **(Table 1).** The decrease in ED is not surprising given the inclusion of the AOS engine in the mouthpiece of the device, which increases the surface area for powder deposition within the device. In addition, there may be eddies in airflow created post-orifice that may trap powder in the corners of the dispersion chamber. Nonetheless, significant increases in the mass of drug depositing on stage 3 to filter (S3-F), and on stage 4 to filter (S4-F) are observed with the AOS designs as compared to RS01. The fine particle dose on stage 3 to filter ($FPD_{S3-F}$) for the RS01 device was 23.9 mg. This represents 32% of the label claim. The S3-F stage grouping is consistent with the observed *in vivo* total lung dose of 34% of the label claim observed clinically (Weers et al: J Aerosol Med Pulm Drug Deliv. 2015; 28:268-280). The differences in $FPD_{S3-F}$ is minimal between the prototype designs. The $FPD_{S4-F}$, a metric for small airway delivery is just 13% for the RS01. Small airway delivery is accentuated for the NBP designs as compared to the BP designs. A direct relationship was observed between device resistance in the prototype designs and $FPD_{S4-F}$ pointing to the advantages that the higher resistance NBP designs may bring to maximizing small airway delivery.

**Table 1.** Aerodynamic particle size distributions of indacaterol inhalation powder in various prototype dry powder inhaler designs (N=2 replicates, except for NBP 3.2 SC and RS01, where N=4). All of the prototype designs had a bead diameter, $D_b$, of 4 mm; a chamber diameter, $D_d$, of 5.9 mm, and a chamber length, L, of 8.4 mm. All prototype designs utilized an RS01 (low resistance) base and maintained the external dimensions of the RS01 mouthpiece.

| Device | $D_i$ (mm) | Retaining Member | Outlet diameter (mm) | R * | Impactor ED (µg) | $FPD_{S3-F}$ (µg) | $FPD_{S4-F}$ (µg) |
|---|---|---|---|---|---|---|---|
| | | | | | Mean | Mean | Mean |
| BP 3.2 SC | 3.2 | single | 7.5 | 0.114 | 60.8 | 28.5 | 18.4 |
| BP 2.8 CC | 2.8 | cross | 7.5 | 0.140 | 58.4 | 28.6 | 19.9 |
| BP 2.8 SC | 2.8 | single | 7.5 | 0.144 | 57.8 | 28.0 | 19.5 |
| NBP 3.2 SC | 3.2 | single | 10.75 | 0.174 | 57.2 | 28.7 | 21.3 |
| NBP 2.8 SC | 2.8 | single | 10.75 | 0.228 | 56.3 | 27.7 | 21.9 |
| RS01 | --- | --- | 11 | 0.100 | 61.9 | 23.9 | 13.4 |

*Units for device resistance are cm $H_2O^{0.5} L^{-1}$ min

Whereas deposition on S3-F is consistent with the total lung dose, deposition on S4-F provides an *in vitro* metric for small airway delivery. Overall, the prototype NBP 3.2 SC design exhibited a 1.2-fold improvement in S3-F deposition, and a 1.6-fold improvement in S4-F deposition relative to the RS01. These results indicate that the additional dispersion elements further disperse the powder agglomerates enabling more effective delivery to the small airways.

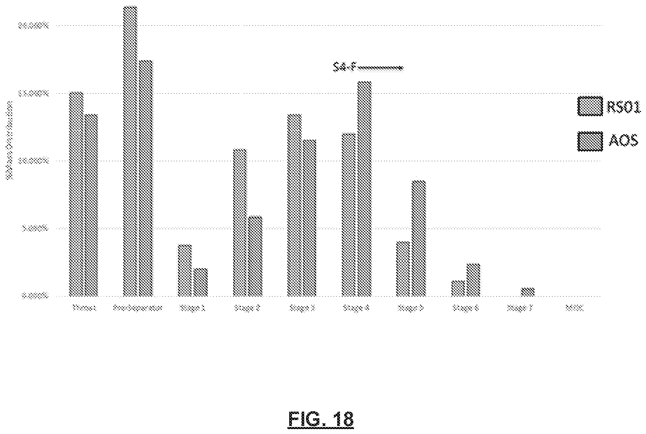

The differences in the APSD for the prototype NBP 3.2 design relative to the RS01 are illustrated in **FIG. 18.** Deposition of indacaterol was significantly decreased on stages 1 to 3 for the NBP 3.2 SC relative to the RS01 but was significantly increased for stages 4 to 7. As discussed, this shift in the pattern of deposition on the NGI to smaller impaction parameters is expected to increase aerosol delivery to the small airways with standard lactose blends like Arcapta.

In the second study, additional AOS design features were explored **(Table 2).**

**Table 2.** APSD of indacaterol inhalation powder in various prototype dry powder inhaler designs (N=3 replicates). All of the prototype designs tested had a bead diameter, $D_b$, of 4 mm; a chamber diameter, $D_d$, of 5.9 mm, and a chamber length, L, of 10 mm. Oval outlets have a 16x11 mm outlet size. All prototype designs utilized an RS01 (low resistance) base and maintained the external dimensions of the RS01 mouthpiece.

| Device | $D_i$ (mm) | Retaining Member | Outlet diameter (mm) | R* | Impactor ED (µg) Mean | FPD$_{S3-F}$ (µg) Mean | FPD$_{S4-F}$ (µg) Mean |
|---|---|---|---|---|---|---|---|
| BP 3.4 SC | 3.4 | single | 9.8 | 0.120 | 57.8 | 26.20 | 15.98 |
| BP 3.2 SC | 3.2 | single | 9.8 | 0.124 | 56.3 | 27.03 | 17.26 |
| NBP 3.4 SC | 3.4 | single | 11 | 0.160 | 55.7 | 26.96 | 18.89 |
| NBP 3.4 SO | 3.4 | single | 16 x 11 | 0.163 | 56.6 | 26.28 | 18.52 |
| NBP 3.2 SO | 3.2 | single | 16 x 11 | 0.175 | 51.7 | 25.14 | 18.63 |
| NBP 3.4 CC | 3.4 | cross | 11 | 0.177 | 54.0 | 25.71 | 18.44 |
| NBP 3.2 SC | 3.2 | single | 11 | 0.178 | 58.6 | 27.37 | 19.16 |
| NBP 3.4 CO | 3.4 | cross | 16 x 11 | 0.188 | 54.1 | 25.95 | 19.27 |
| NBP 3.2 CC | 3.2 | cross | 11 | 0.188 | 55.0 | 27.30 | 19.64 |
| NBP 3.2 CO | 3.2 | cross | 16 x 11 | 0.198 | 50.1 | 26.14 | 19.88 |
| RS01 | --- | --- | 11 | 0.100 | 61.7 | 23.19 | 12.08 |

*Units for device resistance are cm $H_2O^{0.5} L^{-1}$ min

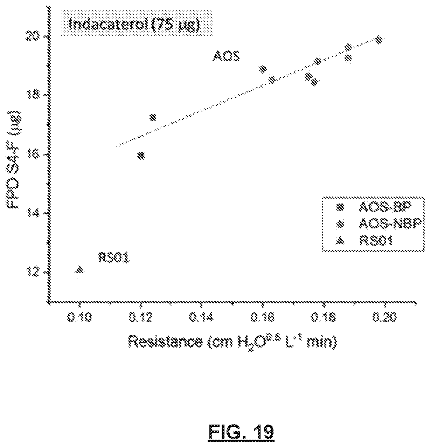

The results are consistent with the first study in that the FPD$_{S4-F}$ increased with increases in device resistance. This is illustrated graphically in **FIG. 19**. The improved aerosol performance at high resistance favors the NBP designs.

**Example 2. Impact of dry powder inhaler design on the aerodynamic particle size distribution of vardenafil inhalation powder**

Vardenafil hydrochloride is a potent vasodilator with potential for the treatment of patients with pulmonary arterial hypertension (PAH). Delivery of the drug via inhalation to the small airways is critical for optimizing targeting of drug into the pre-capillary region of the pulmonary vasculature.

Three vardenafil inhalation powder formulations from a laboratory-scale formulation screening study were selected for assessment in three prototype AOS dry powder inhalers. The compositions of the three formulations on an anhydrous basis are detailed in **Table 3.**

**Table 3.** Compositions of selected vardenafil inhalation powder compositions (anhydrous basis)[1]

| Formulation | Vardenafil HCl (%/w/w) | Coarse Lactose (%w/w) | Fine Lactose (%w/w) | Magnesium stearate (%w/w) |
|---|---|---|---|---|
| HQ00005 | 2.0 | 93 | 5.0 | 0.0 |
| HQ00006 | 2.0 | 90.5 | 7.5 | 0.0 |
| HQ00009 | 2.0 | 92 | 5.0 | 1.0 |
| [1] The vardenafil content in the formulations was 1.77%, for a nominal vardenafil dose of 531 µg (30 mg fill mass) | | | | |

The three formulations differ in the percentage of fine lactose and in the addition of a force control agent (magnesium stearate). The addition of fine lactose and/or the force control agent improves dispersibility of drug from the coarse carrier particles.

The lactose blends were prepared by high shear blending with a Diosna high shear mixer. The powder blends were filled into size 3 hypromellose capsules with a Quantos automated capsule filling machine to a fill mass of 30 mg. Aerosol performance was assessed in three prototype AOS devices in comparison with the RS01.

APSDs were determined on a Next Generation Impactor operated at a 4 kPa pressure drop with an inhaled volume of 4 L, as described in **Example 1.** Vardenafil was quantitated by HPLC using a method that was adapted from the USP vardenafil method. Stages were coated with a glycerol solution.

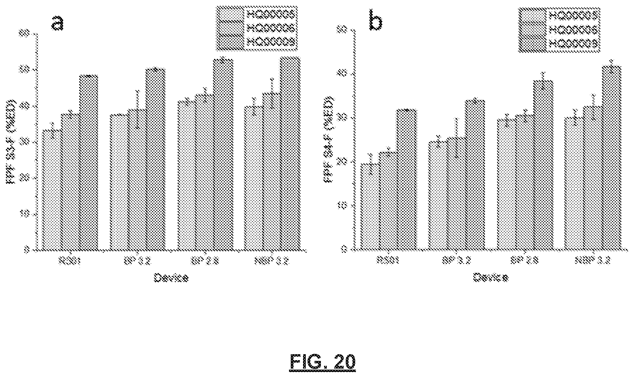

The NGI data are compiled in **Table 4.** Aerosol performance results for the various combinations of vardenafil formulations and prototype devices is also presented in **FIG. 20.** The rank ordering of the vardenafil formulations is consistent for each of the four devices. That is, aerosol performance increased in the following order: 5% lactose fines (HQ00005) < 7.5% lactose fines (HQ00006) < 5% lactose fines + 1% FCA (HQ00009).

The rank ordering of the prototype AOS devices with the various vardenafil formulations is consistent with previous aerosol performance results obtained for these devices with Arcapta (indacaterol inhalation powder) **(Example 1).** That is, aerosol performance increased in the following order: RS01 << BP 3.2 SC < BP 2.8 SC < NBP 3.2 SC.

Improved delivery to the small airways is critical for vardenafil inhalation powder, as targeting into small airways enables direct delivery to arterioles in the pre-capillary region of the pulmonary circulation.

**Table 4.** APSD of vardenafil inhalation powder in various prototype dry powder inhaler designs (N=3 replicates). All of the prototype designs tested had a bead diameter, $D_b$, of 4 mm; a chamber diameter, $D_d$, of 5.9 mm, and a chamber length, L, of 8.4 mm. All prototype designs utilized an RS01 (low resistance) base and maintained the external dimensions of the RS01 mouthpiece.

| Formulation | Device | % Impactor ED (SD) | FPD$_{S3-F}$ ($\mu$g) (SD) | FPF$_{S53-F}$ (%ED) (SD) | FPD$_{S4-F}$ ($\mu$g) (SD) | FPF$_{S4-F}$ (%ED) (SD) |
|---|---|---|---|---|---|---|
| HQ00005 | BP 2.8 SC | 89.6 (1.2) | 198.0 (7.1) | 41.2 (0.9) | 141.8 (8.7) | 29.5 (1.4) |
| | BP 3.2 SC | 89.7 (1.1) | 181.0 (2.6) | 37.6 (0.1) | 118.4 (4.3) | 24.6 (1.2) |
| | NBP 3.2 SC | 85.6 (2.2) | 184.3 (15.2) | 39.8 (2.3) | 138.5 (11.4) | 30.1 (1.7) |
| | RS01 | 90.2 (2.0) | 161.3 (6.7) | 33.3 (2.1) | 93.8 (9.1) | 19.4 (2.3) |
| HQ00006 | BP 2.8 SC | 82.9 (2.6) | 192.1 (14.0) | 43.1 (1.8) | 136.0 (10.5) | 30.5 (1.4) |
| | BP 3.2 SC | 81.1 (6.0) | 170.6 (34.9) | 39.0 (5.1) | 111.4 (27.4) | 25.4 (4.4) |
| | NBP 3.2 SC | 83.3 (4.0) | 195.4 (25.7) | 43.5 (4.0) | 146.0 (18.3) | 32.5 (2.8) |
| | RS01 | 89.9 (3.5) | 181.9 (11.6) | 37.7 (1.0) | 107.2 (8.4) | 22.2 (0.9) |
| HQ00009 | BP 2.8 SC | 78.7 (0.8) | 223.1 (5.7) | 52.8 (0.8) | 162.5 (9.5) | 38.4 (1.8) |
| | BP 3.2 SC | 79.1 (1.0) | 213.4 (1.1) | 50.2 (0.4) | 144.3 (0.8) | 34.0 (0.6) |
| | NBP 3.2 SC | 75.9 (2.3) | 217.5 (6.5) | 53.3 (0.0) | 170.1 (0.3) | 41.7 (1.4) |
| | RS01 | 81.5 (1.4) | 211.1 (2.7) | 48.3 (0.2) | 139.3 (3.4) | 31.8 (0.2) |

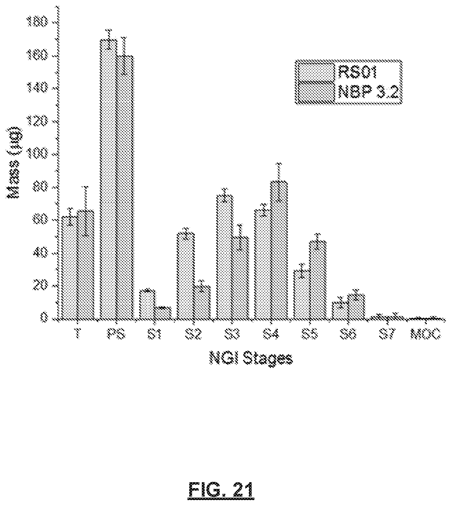

The increases in peripheral deposition are reflected in the shift in the maximum in the APSD from Stage 3 to Stage 4 with the transition from the RS01 to prototype AOS DPIs **(FIG. 21). FIG. 21** is a plot comparing the aerodynamic particle size distributions obtained for a 2.0% w/w vardenafil hydrochloride with 7.5% w/w lactose fines [Formulation HQ00006] administered with the RS01 DPI and an experimental prototype AOS DPI with a 3.2 mm inlet diameter and no bypass (NBP 3.2 SC). APSD measurements were conducted with a NGI operated at a 4 kPa pressure drop, an inhaled volume of 4 L, and a single actuation. The acronyms on the abscissa have the following meanings: T (induction port or 'throat'); PS (pre-separator); S1 to S7 (stages 1 to 7); MOC (micro-orifice collector or filter). Inspection of **FIG. 21** reveals that increased deposition is observed for the RS01 device on stages 1 to 3, while the prototype device exhibits greater deposition on stages 4 to 6. The shift of particles into later stages in the NGI is expected to result in increased peripheral deposition in the small airways *in vivo.*

The mass median aerodynamic diameters for the various formulation and device combinations are detailed in **Table 5.** The MMAD values are fairly comparable for the various devices indicating similar degrees of powder dispersion, despite the fact that the prototype devices are actuated at significantly lower flow rates. The differences in the pattern of deposition observed for the various inhalers is the result of decreased inertial impaction parameters that depend on both the aerodynamic size and flow rate. The improved performance of prototype devices at higher resistance values is also reflective of decreased impaction parameters with increased resistance (comparable aerodynamic size, lower flow rates). This points to the criticality of higher resistance AOS DPIs for improving delivery to the small airways.

**Table 5.** Mass median aerodynamic diameters for vardenafil hydrochloride lactose blends delivered with the RS01 and three prototype dry powder inhalers.

| Formulation | Device | MMAD ($\mu$m) $\Delta$P = 4 kPa | MMAD ($\mu$m) $\Delta$P = 2 kPa |
|---|---|---|---|
| HQ00005 | 2.8 BP | 2.53 | |
| | 3.2 BP | 2.57 | |
| | 3.2 NBP | 2.70 | |
| | RS01 | 2.75 | |
| HQ00006 | 2.8 BP | 2.62 | |
| | 3.2 BP | 2.69 | 3.05 |
| | 3.2 NBP | 2.77 | |
| | RS01 | 2.77 | 3.22 |
| HQ00009 | 2.8 BP | 2.20 | |
| | 3.2 BP | 2.26 | |
| | 3.2 NBP | 2.28 | |
| | RS01 | 2.14 | |

**Example 3. Flow rate dependence of vardenafil inhalation powder in RS01 and prototype devices**

Bypassing deposition in the upper respiratory tract is expected to lead to reductions in the impact that inspiratory flow rate has on particle deposition in the lungs.

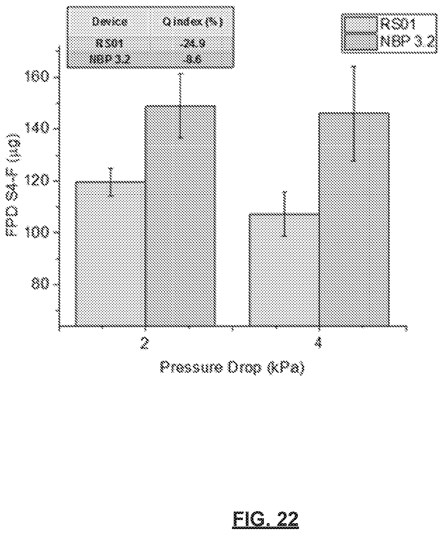

The flow rate dependence of the vardenafil formulation comprising 7.5% fine lactose (HQ00006) in the RS01 and AOS NBP 3.2 SC dry powder inhalers at 4 kPa and 2 kPa pressure drops is plotted in **FIG. 22.** At the 4 kPa pressure drop, $FPD_{S4-F}$ is increased by about 1.4-fold by inclusion of the prototype engine in the RS01 mouthpiece.

The magnitude of the flow rate dependence can be assessed using a metric termed the Q *index* (Weers and Clark, Pharm Res. 2017, 34:507-528). The Q *index* is derived from a plot of $FPD_{S4-F}$ versus pressure drop. It represents the percent difference in $FPD_{S4-F}$ between pressure drops of 6 kPa and 1 kPa normalized by the higher of the two $FPD_{S4-F}$ values, viz:

$$Qindex = \left[\frac{FPD_{6kPa} - FPD_{1kPa}}{FPD_{higher}}\right] x100 \qquad (1)$$

This range of pressure drops encompasses what most patients achieve when inhaling comfortably with a portable DPI. The sign of the Q *index* indicates whether lung delivery is increased with pressure drop (positive flow rate dependence) or decreased with pressure drop (negative flow rate dependence). Low flow rate dependence is defined as having a Q *index* between 0% and 15%, medium flow rate dependence between 15% and 40%, and high flow rate dependence greater than 40%. In the present study the Q *index* is obtained by extrapolation of a linear regression of the data at 4 kPa and 2 kPa. Based on the results in **FIG. 22,** delivery of HQ00006 with the prototype NBP AOS 3.2 dry powder inhaler leads to low flow rate dependence, while delivery with the RS01 has a medium flow rate dependence. The decrease in Q *index* observed with the prototype DPI relative to the RS01 is consistent with the observation that increasing lung delivery efficiency with portable dry powder inhalers decreases flow rate dependence (Weers and Clark, Pharm Res. 2017, 34:507-528, the entire contents of which is hereby incorporated by reference). The negative flow rate dependence suggests that the impact of flow rate on the impaction parameter is greater than the impact of changes in APSD (i.e., the formulations disperse quite well in both devices). Similar results have been observed with indacaterol formulations in the related Concept1 DPI (Weers, 2015).

Specific details are given in the description to provide a thorough understanding of example configurations (including implementations). However, configurations may be practiced without these specific details, but the resulting subject-matter is according to the invention only if it falls within the scope of the claims. For example, well-known processes, structures, and techniques have been shown without unnecessary detail in order to avoid obscuring the configurations. This description provides example configurations only, and does not limit the scope, applicability, or configurations of the claims. Rather, the preceding description of the configurations will provide those skilled in the art with an enabling description for implementing described techniques. Various changes may be made in the function and arrangement of elements without departing from the scope of the invention as defined by the claims.

Also, configurations may be described as a process (not according to the invention) that is depicted as a flow diagram or block diagram. Although each may describe the operations as a sequential process, many of the operations may be performed in parallel or concurrently. In addition, the order of the operations may be rearranged. A process may have additional steps not included in the figure.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

The invention has now been described in detail for the purposes of clarity and understanding. However, it will be appreciated that certain changes and modifications may be practiced within the scope of this invention as defined by the claims.

**Claims**

1. A unit dose dry powder inhaler (400), comprising:

    an inhaler base (428) defining a capsule seat (430) that is configured to hold a capsule (434) containing a powdered medicament, the inhaler base (428) comprising at least one primary air intake (436) that is in fluid communication with the capsule seat (430), wherein the at least one primary air intake (436) is configured to, in use, draw air into the capsule seat (430) so as to generate rapid capsule precession and centrifugal forces that fluidize and disperse powder agglomerates;
    a grid (412) positioned between the inhaler base (428) and a mouthpiece, wherein the inhaler is structured such that, in use, entrained powder is drawn through the grid (412) resulting in dispersion of powder agglomerates via impaction forces with the static grid;
    an inlet funnel (404) positioned between the grid and a dispersion chamber (406), wherein the inlet funnel (404) tapers to a small inlet orifice (410) into the dispersion chamber (406) resulting, in use, in dispersion of powder agglomerates via shear forces generated at the orifice (410);
    the dispersion chamber (406),
    that is adapted to entrain powder from the inlet funnel (404), the dispersion chamber (406) holding an actuator (416) that is configured to oscillate within the dispersion chamber (406) during inhalation resulting in dispersion of powder agglomerates by dynamic impaction with the actuator (416) and increased turbulence within the dispersion chamber (406); and
    the mouthpiece (402), that includes an outlet funnel (408) through which air and aerosolized medicament exit the inhaler (400) to be delivered to a patient, wherein the outlet funnel (408) is tapered to, in use, reduce or minimize particle velocity and turbulence at an exit of the mouthpiece to the patient,
    **characterised in that**:

    the grid (412) has a diameter of between 10 mm and 20 mm;
    the diameter of the inlet funnel (404) at its grid side matches the diameter of the grid; and
    a diameter of the orifice (410) is between 2.8 mm and 3.4 mm.

2. The unit dose dry powder inhaler of claim 1, wherein:
    the inlet funnel and the outlet funnel have a conical frustum shape, or wherein:
    the inhaler includes air bypass channels to modulate device resistance.

3. The unit dose dry powder inhaler of claim 1, wherein:
    the dry powder inhaler does not include air bypass channels to modulate device resistance.

4. The unit dose dry powder inhaler of claim 1, further comprising:
    a retaining member coupled with an outlet end of the dispersion chamber.

5. A unit dose dry powder inhaler according to claim 1, further comprising:

at least one piercing member that is configured to pierce the capsule upon actuation;

wherein the at least one primary air intake is configured to draw air into the capsule seat upon piercing the capsule so as to generate rapid capsule precession and centrifugal forces that fluidize and disperse powder agglomerates;

wherein the dispersion chamber comprises a step increase in diameter relative to the small inlet orifice.

6. The unit dose dry powder inhaler of claim 5, wherein:
the inlet funnel and the dispersion chamber are formed as a single unit that is configured to couple with the outlet funnel.

7. The unit dose dry powder inhaler of claim 6, further comprising:
an inhaler body comprising the outlet funnel, wherein the single unit is configured to be inserted within the inhaler body to couple the dispersion chamber with the outlet funnel.

8. The unit dose dry powder inhaler of claim 7, wherein:
the inhaler body defines a receptacle that is configured to receive a flange of the grid after the single unit is inserted within the inhaler body such that the grid is positioned between the inlet funnel and the base.

9. The unit dose dry powder inhaler of claim 5, wherein:
the grid comprises a rim that is configured to receive at least a portion of an upstream end of the inlet funnel.


**Patentansprüche**

1. Trockenpulverinhalator mit Einheitsdosis (400), der Folgendes umfasst:
einen Inhalatorgrundkörper (428), der eine Kapselaufnahme (430) definiert, die zum Halten einer Kapsel (434) konfiguriert ist, die ein pulverförmiges Medikament enthält, wobei der Inhalatorgrundkörper (428) Folgendes umfasst:

mindestens einen primären Lufteinlass (436), der in Fluidverbindung mit der Kapselaufnahme (430) steht, wobei der mindestens eine primäre Lufteinlass (436) konfiguriert ist, um bei Verwendung Luft in die Kapselaufnahme (430) zu ziehen, um eine schnelle Kreiselbewegung der Kapsel und Zentrifugalkräfte zu erzeugen, die Pulveragglomerate fluidisieren und dispergieren;

ein Gitter (412), das zwischen dem Inhalatorgrundkörper (428) und einem Mundstück positioniert ist, wobei der Inhalator derart strukturiert ist, dass bei Verwendung mitgeführtes Pulver durch das Gitter (412) gezogen wird, was zur Dispersion von Pulveragglomeraten durch Aufschlagkräfte mit dem statischen Gitter führt;

einen Einlasstrichter (404), der zwischen dem Gitter und einer Dispersionskammer (406) positioniert ist, wobei sich der Einlasstrichter (404) zu einer kleinen Einlassöffnung (410) in die Dispersionskammer (406) verjüngt, was bei Verwendung zur Dispersion von Pulveragglomeraten durch Scherkräfte führt, die an der Öffnung (410) erzeugt werden;

die Dispersionskammer (406), die zum Mitführen von Pulver aus dem Einlasstrichter (404) geeignet ist, wobei die Dispersionskammer (406) einen Aktor (416) hält, der konfiguriert ist, um innerhalb der Dispersionskammer (406) während einer Inhalation zu schwingen, was zur Dispersion von Pulveragglomeraten durch dynamischen Aufschlag mit dem Aktor (416) und erhöhter Verwirbelung innerhalb der Dispersionskammer (406) führt; und

das Mundstück (402), das einen Auslasstrichter (408) einschließt, durch den Luft und zerstäubtes Medikament aus dem Inhalator (400) austreten, um an den Patienten abgegeben zu werden, wobei sich der Auslasstrichter (408) verjüngt, um bei Verwendung die Partikelgeschwindigkeit und Verwirbelung an einem Ausgang des Mundstücks zu dem Patienten zu verringern oder zu minimieren,

**dadurch gekennzeichnet, dass**:

das Gitter (412) einen Durchmesser von zwischen 10 mm und 20 mm aufweist;
der Durchmesser des Einlasstrichters (404) an seiner Gitterseite dem Durchmesser des Gitters entspricht; und
ein Durchmesser der Öffnung (410) zwischen 2,8 mm und 3,4 mm beträgt.

2. Trockenpulverinhalator mit Einheitsdosis nach Anspruch 1, wobei:
der Einlasstrichter und der Auslasstrichter eine Kegelstumpfform aufweisen oder wobei:
der Inhalator Luftumleitungskanäle einschließt, um den Vorrichtungswiderstand zu modulieren.

**3.** Trockenpulverinhalator mit Einheitsdosis nach Anspruch 1, wobei:
der Trockenpulverinhalator keine Luftumleitungskanäle einschließt, um den Vorrichtungswiderstand zu modulieren.

**4.** Trockenpulverinhalator mit Einheitsdosis nach Anspruch 1, der weiter Folgendes umfasst:
ein Rückhalteelement, das mit einem Auslassende der Dispersionskammer verbunden ist.

**5.** Trockenpulverinhalator mit Einheitsdosis nach Anspruch 1, der weiter Folgendes umfasst:

mindestens ein Durchstechelement, das konfiguriert ist, um die Kapsel bei Betätigung zu durchstechen;
wobei der mindestens eine primäre Lufteinlass konfiguriert ist, um Luft in die Kapselaufnahme beim Durchstechen der Kapsel zu ziehen, um eine schnelle Kreiselbewegung der Kapsel und Zentrifugalkräfte zu erzeugen, die Pulveragglomerate fluidisieren und dispergieren;
wobei die Dispersionskammer eine stufenweise Erhöhung des Durchmessers relativ zu der kleinen Einlassöffnung umfasst.

**6.** Trockenpulverinhalator mit Einheitsdosis nach Anspruch 5, wobei:
der Einlasstrichter und die Dispersionskammer als eine Einzeleinheit ausgebildet sind, die zur Verbindung mit dem Auslasstrichter konfiguriert ist.

**7.** Trockenpulverinhalator mit Einheitsdosis nach Anspruch 6, der weiter Folgendes umfasst:
einen Inhalatorkörper, der den Auslasstrichter umfasst, wobei die Einzeleinheit konfiguriert ist, um innerhalb des Inhalatorkörpers eingeführt zu werden, um die Dispersionskammer mit dem Auslasstrichter zu verbinden.

**8.** Trockenpulverinhalator mit Einheitsdosis nach Anspruch 7, wobei:
der Inhalatorkörper eine Aufnahmefassung definiert, die zur Aufnahme eines Flansches des Gitters nach der Einführung der Einzeleinheit innerhalb des Inhalatorkörpers konfiguriert ist, sodass das Gitter zwischen dem Einlasstrichter und dem Grundkörper positioniert ist.

**9.** Trockenpulverinhalator mit Einheitsdosis nach Anspruch 5, wobei:
das Gitter eine Einfassung umfasst, die zur Aufnahme von zumindest einem Teil eines vorgelagerten Endes des Einlasstrichters konfiguriert ist.

## Revendications

**1.** Inhalateur de poudre sèche à dose unitaire (400), comprenant :

une base d'inhalateur (428) définissant une embase de capsule (430) qui est configurée pour tenir une capsule (434) contenant un médicament pulvérisé, la base d'inhalateur (428) comprenant au moins une prise d'air primaire (436) qui est en communication fluidique avec l'embase de capsule (430), dans lequel la au moins une prise d'air (436) est configurée pour, en emploi, tirer de l'air dans l'embase de capsule (430) de manière à produire une précession rapide de la capsule et des forces centrifuges qui fluidisent et dispersent des agglomérats de poudre ;
une grille (412) positionnée entre la base d'inhalateur (428) et une embouchure, où l'inhalateur est structuré de manière telle qu'en emploi, de la poudre entraînée est tirée à travers la grille (412) résultant en la dispersion d'agglomérats de poudre via les forces d'impact à travers la grille statique ;
un entonnoir d'admission (404) positionné entre la grille et une chambre de dispersion (406), dans lequel l'entonnoir d'admission (404) rétrécit en un petit orifice d'admission (410) dans la chambre de dispersion (406) résultant, en emploi, en la dispersion d'agglomérats de poudre via les forces de cisaillement produites à l'orifice (410) ;
la chambre de dispersion (406), qui est adaptée pour entraîner la poudre de l'entonnoir d'admission (404), la chambre de dispersion (406) tenant un actionneur (416) qui est configuré pour osciller à l'intérieur de la chambre de dispersion (406) durant l'inhalation résultant en la dispersion d'agglomérats de poudre par impact dynamique avec l'actionneur (416) et en une turbulence accrue à l'intérieur de la chambre de dispersion (406) ; et
l'embouchure (402), qui comprend un entonnoir de sortie (408) à travers lequel de l'air et un médicament aérosolisé sortent de l'inhalateur (400) pour être délivrés à un patient, dans lequel l'entonnoir de sortie (408) rétrécit pour, en emploi, réduire ou minimiser la vitesse des particules et la turbulence à une sortie de l'embouchure au patient,

**caractérisé en ce que** :

la grille (412) a un diamètre d'entre 10 mm et 20 mm ;
le diamètre de l'entonnoir d'admission (404) à son côté grille correspond au diamètre de la grille ; et
un diamètre de l'orifice (410) est d'entre 2,8 mm et 3,4 mm.

2. Inhalateur de poudre sèche à dose unitaire selon la revendication 1, dans lequel :
l'entonnoir d'admission et l'entonnoir de sortie ont une forme en cône tronqué, ou dans lequel :
l'inhalateur comprend des canaux de dérivation d'air pour moduler la résistance du dispositif.

3. Inhalateur de poudre sèche à dose unitaire selon la revendication 1, dans lequel :
l'inhalateur de poudre sèche à dose unitaire ne comprend pas de canaux de dérivation d'air pour moduler la résistance du dispositif.

4. Inhalateur de poudre sèche à dose unitaire selon la revendication 1, comprenant en outre :
un membre de retenue couplé à une extrémité de sortie de la chambre de dispersion.

5. Inhalateur de poudre sèche à dose unitaire selon la revendication 1, comprenant en outre :

au moins un membre de perçage qui est configuré pour percer la capsule lors de l'actionnement ;
dans lequel la au moins une prise d'air primaire est configurée pour tirer de l'air dans l'embase de capsule lors du perçage de la capsule de manière à produire une précession rapide de la capsule et des forces centrifuges qui fluidisent et dispersent les agglomérats de poudre ;
dans lequel la chambre de dispersion comprend une augmentation progressive en diamètre par rapport au petit orifice d'admission.

6. Inhalateur de poudre sèche à dose unitaire selon la revendication 5, dans lequel :
l'entonnoir d'admission et la chambre de dispersion sont formés en une unité unique qui est configurée pour se coupler à l'entonnoir de sortie.

7. Inhalateur de poudre sèche à dose unitaire selon la revendication 6, comprenant en outre :
un corps d'inhalateur comprenant l'entonnoir de sortie, dans lequel l'unité unique est configurée pour être introduite dans le corps d'inhalateur pour coupler la chambre de dispersion à l'entonnoir de sortie.

8. Inhalateur de poudre sèche à dose unitaire selon la revendication 7, dans lequel :
le corps d'inhalateur définit un logement qui est configuré pour recevoir une bride de la grille après que l'unité unique a été introduite dans le corps d'inhalateur de telle sorte que la grille est positionnée entre l'entonnoir d'admission et la base.

9. Inhalateur de poudre sèche à dose unitaire selon la revendication 5, dans lequel :
la grille comprend un rebord qui est configuré pour recevoir au moins une partie d'une extrémité en amont de l'entonnoir d'admission.

Disbursed Powder
Emitted From DPF

100

108

106

104

110

116

122

102

120

101

DPF

118

112

114

FIG. 1

200

208

204

216

202

222

206

220

201

224

210

A

A

A-A Cross
Section

FIG. 2

FIG. 3

**FIG. 4**

FIG. 5

305

304

310

303

302

Rectangular Cross
Section

**FIG. 6**

FIG. 7

312

312

**FIG. 8**

EP 3 612 259 B1

FIG 9

$D_b$ = bead diameter

$D_d$ = AOS chamber diameter

$D_i$ = AOS inlet orifice diameter

L = AOS chamber length

FIG. 10

EP 3 612 259 B1

**FIG. 11**

FIG. 12

EP 3 612 259 B1

FIG. 13

EP 3 612 259 B1

**FIG. 14**

FIG. 15

**FIG. 16**

| | |
|---|---|
| 502 | PROVIDING AN INHALER |

| | |
|---|---|
| 504 | INTRODUCING AIR INTO A CAPSULE SEAT OF THE INHALER TO ENTRAIN POWDER WITHIN THE AIR |

| | |
|---|---|
| 506 | GENERATING CENTRIFUGAL FORCES IN THE CAPSULE SEAT TO FLUIDIZE AND DISPERSE POWDERED MEDICAMENT |

| | |
|---|---|
| 508 | DRAWING THE ENTRAINED POWDER THROUGH A GRID SO AS TO IMPACT AT LEAST A PORTION OF THE POWDERED MEDICAMENT AGAINST THE GRID TO DISPERSE THE PORTION OF THE POWDERED MEDICAMENT |

| | |
|---|---|
| 510 | DISPERSING POWDER AGGLOMERATES VIA INCREASING SHEAR FORCES PRODUCED BY AIR FLOWING THROUGH AN INLET FUNNEL AS AIR APPROACHES A DISPERSION CHAMBER |

| | |
|---|---|
| 512 | GENERATING DYNAMIC IMPACTION AND TURBULENT FORCES BY INDUCING AIR TO FLOW THROUGH THE DISPERSION CHAMBER |

| | |
|---|---|
| 514 | REDUCING THE TURBULENT FORCES AND REDUCING VELOCITY OF THE DEAGGREGATED POWDERED MEDICAMENT IN A FLOW FIELD EXITING THE INHALER |

| | |
|---|---|
| 516 | DELIVERING POWDERED AGGLOMERATES TO A PATIENT'S AIRWAY VIA AN OUTLET FUNNEL OF THE INHALER |

500

**FIG. 17**

FIG. 18

FIG. 19

FIG. 20

FIG. 21

**FIG. 22**

**EP 3 612 259 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62486183
- US 20160199598 A1
- US 20150246189
- US 20150314086
- US 7784552 A

### Non-patent literature cited in the description

- **LAVORINI et al.** *CHEST.,* 2017, vol. 151, 1345-1355
- **WEERS et al.** *J Aerosol Med Pulm Drug Deliv.,* 2015, vol. 28, 268-280
- **WEERS ; CLARK.** *Pharm Res.,* 2017, vol. 34, 507-528